# EUROPEAN PATENT SPECIFICATION

(11) **EP 4 219 501 B1**
(45) Date of publication and mention of the grant of the patent: **28.01.2026**
(21) Application number: 21942477.7
(22) Date of filing: 07.06.2021
(51) Int. Cl.: C07D 487/04, C07D 417/14, C07D 417/06, C07J 71/00, C07J 21/00, C07J 43/00, A61K 31/427, A61P 37/06

(54) **COMPOUND CONTAINING 2,4-THIAZOLE RING, PREPARATION METHOD THEREFOR, AND APPLICATION THEREOF**
VERBINDUNG MIT 2,4-THIAZOLRING, HERSTELLUNGSVERFAHREN DAFÜR UND ANWENDUNG DAVON
COMPOSÉ CONTENANT UN CYCLE 2,4-THIAZOLE, SON PROCÉDÉ DE PRÉPARATION ET SON UTILISATION

(30) Priority: 27.05.2021 CN 202110583450
(43) Date of publication of application: 02.08.2023
(73) Proprietor: Shandong University, Jinan, Shandong 250012 (CN)
(72) Inventor: LOU, Hongxiang, Jinan, Shandong 250012 (CN); FANG, Liyuan, Jinan, Shandong 250012 (CN); SUN, Bin, Jinan, Shandong 250012 (CN)
(74) Representative: Lorenz Seidler Gossel Part. mbB
(86) International application number: PCT/CN2021/098707
(87) International publication number: WO 2022/246907

(56) References cited:
- WO-A1-2020/106695
- WO-A2-2019/099977
- CN-A- 111 587 249
- US-A1- 2019 284 149
- TANTAK MUKUND P.; WANG JING; SINGH RAJNISH PRAKASH; KUMAR ANIL; SHAH KAVITA; KUMAR DALIP: "2-(3′-Indolyl)-N-arylthiazole-4-carboxamides: Synthesis and evaluation of antibacterial and anticancer activi", BIOORGANIC & MEDICINAL CHEMISTRY LETTERS, ELSEVIER, AMSTERDAM NL, vol. 25, no. 19, 6 August 2015 (2015-08-06), Amsterdam NL , pages 4225 - 4231, XP029264250, ISSN: 0960-894X, DOI: 10.1016/j.bmcl.2015.07.105
- GUO HUIJUAN; RISCHER MAJA; SPERFELD MARTIN; WEIGEL CHRISTIANE; MENZEL KLAUS DIETER; CLARDY JON; BEEMELMANNS CHRISTINE: "Natural products and morphogenic activity of γ-Proteobacteria associated with the marine hydroid polypHydractinia echinata", BIOORGANIC & MEDICINAL CHEMISTRY, ELSEVIER, AMSTERDAM, NL, vol. 25, no. 22, 1 July 2017 (2017-07-01), AMSTERDAM, NL, pages 6088 - 6097, XP085235306, ISSN: 0968-0896, DOI: 10.1016/j.bmc.2017.06.053

## Description

### TECHNICAL FIELD

The present application relates to a field of organic synthesis and pharmaceutical chemistry, in particular to compounds containing 2,4-thiazole ring, method for preparing same and use thereof.

### BACKGROUND

Any discussion of the prior art throughout the specification should not be taken as an admission that such prior art is widely known or forms part of the common general knowledge in the art.

Autoimmune disease is a disease in which T and B cells are over-activated in self-reaction, and cause damage to their own tissues and organs as a result of an immune response to their own antigens, such as systemic lupus erythematosus (SLE) and psoriasis. Epidemiological surveys show that there are millions of patients with SLE in China. At present, there are still no new chemical drugs for such autoimmune diseases worldwide. Clinical treatment is mainly based on the use of glucocorticoids in combination with non-specific anti-inflammatory and immunosuppressive drugs, which delays the progress of the disease to a certain extent, but long-term use will cause the decline of patients' immune function and cause a variety of complications.

Studies have proved that B lymphocytes and T lymphocytes play an important role in autoimmune diseases. It is an important topic in medical and pharmaceutical research to develop new drugs that can inhibit the proliferation and activation of immune cells and reduce the abnormal immune response of the body to treat autoimmune diseases.

Aryl hydrocarbon receptor (AhR) is a ligand-activated transcription factor expressed mainly in the nucleus and can be activated by a range of compounds, such as the carcinogen 2,3,7,8-tetrachlorodibenzo-p-dioxin (TCDD), which has the property of promoting tumor growth and activating immune cells when activated by agonists such as TCDD. Studies have shown that AhR is a key transcription factor of T helper 22 (Th22) cell and plays an important role in immune response, thus AhR receptor has great potential in the treatment of autoimmune diseases.

In the absence of ligands, AhR is in the cytoplasm and exists as part of a protein complex consisting of heat shock protein (HSP) 90, p23, and AhR. Upon binding of a ligand such as TCDD, the AhR complex is activated and translocated to the nucleus, where AhR is released from chaperone protein and interacts with arylhydroarbon nuclear translocator (ARNT). Chaperone protein protects AhR from protein hydrolysis and retain a structure conducive to ligand binding. AhR-ARNT heterodimer is associated with signaling factors (e.g. chromatin remodeling factors, histone acetyltransferase and transcription factors), and ultimately binds to distal regulatory elements (DREs) or aryl hydrocarbon response elements (AHREs) to facilitate transcriptional regulation. Designing AhR-targeted immunomodulators is of great significance for the treatment of autoimmune diseases.

In the prior art, WO 2019/099977 A1 and CN 111 587 249 A disclose thiazole-carboxylic acid derivatives having an indolyl carbonyl group connected to the thiazole. The compounds are disclosed as stimularoty to the immune system.

Compounds of a similar structure are also disclosed in US 2019/284149 A1, WO 2020/106695 A1, Tantak Mukund P., et al., Bioorganic & Medicinal Chemistry Letters, 25(19), pp. 4225-4231, and Guo Huijuan, et al., Bioorganic & Medicinal Chemistry, 25(22), pp. 6088-6097.

### SUMMARY

The present application provides compounds containing 2,4-thiazole ring in the structure thereof and pharmaceutically acceptable salts thereof, wherein the compounds have less toxic and side effects and have an inhibitory effect on the activity of immune cells.

In particular, the present application provides the following technical features, one or more of which constitute a technical solution for the present application.

In a first aspect of the present application, the present application provides a compound containing a 2,4-thiazole ring or a pharmaceutically acceptable salt thereof, the compound having a structure of Formula X: 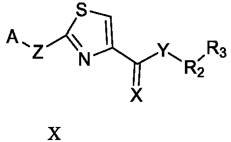
wherein A is a struture of pyrazolo pyrimidine or indole;
and the compound conforms to a structure of Formula X₁: 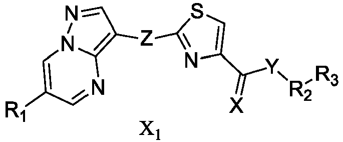
   Z is none or carbonyl; X is oxygen or sulfur; Y is -O-, -NH- or 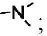
   R₁ is hydrogen or C₁-C₆ alkyl;R₂ is selected from C₁-C₃ alkyl, C₅-C₁₅ alkenyl, alkynyl, 5-10 membered heterocyclyl, C₆-C₁₂ aryl, 5-12 membered heteroaryl, sterol group and 5-10 membered cycloalkyl; Y and R₂ are directly connected, or Y and R₂ are connected to form a ring;
   R₃ is selected from hydrogen, halogen, amino, hydroxyl, acetyl, 3-10 membered heterocyclyl, C₆-C₁₂ aryl, 5-12 membered heteroaryl, 3-10 membered cycloalkyl, ester group, carboxyl, trihalomethyl and adamantyl;
   R₂ or R₃ is unsubstituted, or is substituted by one or more groups selected from C₁-C₆ alkyl, hydroxyl, halogen, trihalomethyl, carboxyl and phenyl;
   wherein R₃ is not hydrogen when R₂ is C₁-C₃ alkyl.

And, in the compounds of Formula X₂, R₃ is not hydroxyl when R₂ is alkyl.

Inventors have found that certain compounds tend to have severe toxicity and side effects while having an inhibitory effect on the activity of immune cells, such as in some embodiments where R₂ is C₁-C₃ alkyl and R₃ is hydrogen, and, in the case of the structure of Formula X₂, where R₂ is alkyl and R₃ is hydroxyl. However, the compounds in the present application have no obvious toxicity at the test concentration, and have high safety index, good oral bioavailability and good developability.

In the present application, the term "C₁-C₆ alkyl" refers to a straight-chain, saturated hydrocarbon radical containing 1 to 6 carbon atoms, including, without limitation, methyl, ethyl, propyl, etc.

The term "C₅-C₁₅ alkenyl" refers to a straight-chain or branched hydrocarbon radical with one or more double bonds and containing 5 to 15 carbon atoms.

The term "3-10 membered heterocyclyl" refers to a saturated or partially saturated cyclic group having 3-10 ring atoms of which 1-3 are heteroatoms selected from the group consisting of nitrogen, oxygen and S(O)ₘ (where m is an integer from 0 to 2), the remaining ring atoms being carbon atoms; for example, propylene oxide, tetrahydrofuranyl, pyrrolidinyl, tetrahydropyranyl, piperidinyl, piperazinyl, morpholinyl, thiomorpholinyl, homopiperazinyl, etc.

The term "C₆-C₁₂ aryl" refers to an aromatic ring group containing 6-10 ring atoms, but without heteroatoms in the ring atoms, such as phenyl, naphthyl, biphenyl, etc.

The term "5-12 membered heteroaryl" refers to an aromatic ring group of 5-12 ring atoms containing 1-4 ring heteroatoms. The heteroatom each independently selected from nitrogen, oxygen or sulfur. The heteroaryl may be a monocyclic heteroaryl having 5-7 ring atoms or a bicyclic heteroaryl having 7-12 ring atoms. It is sufficient that one of the bicyclic heteroaryl rings is a heteroaryl, and the other may be an aromatic or non-aromatic ring, with or without a heteroatom. In addition, bicyclic heteroaryl may be a fused ring structure in which two heterocycles share a common ring edge, or in which the two heterocycles are joined directly, such as by a single bond. Examples of heteroaryl groups include, but are not limited to, pyrrolyl, pyrazolyl, imidazolyl, triazolyl, oxazolyl, pyridyl, pyrimidyl, furyl, thienyl, isoxazolyl, indolyl, etc.

The term "sterol group" refers to a group of perhydrocyclopentanophenanthrene derivatives fused by three cyclohexane and one cyclopentane, such as sitosterol group, cholesterol group, ergosterol group, solasodine group and protodioscin group etc.

The term "3-10-membered cycloalkyl" means a group containing one or more saturated and/or partially saturated rings, all of which are carbon atoms, comprising from 3 to 10 carbon atoms; for example, cyclopropyl, cyclobutyl, cyclopentyl, cyclohexyl, cyclohexenyl, cycloheptenyl, cycloheptatrienyl, adamantyl, etc.

The term "halogen" refers to fluorine, chlorine, bromine or iodine.

The term "trihalomethyl" refers to methyl substituted with three same or different halo atoms, such as trifluoromethyl, etc.

In some embodiments in the present application, the pharmaceutically acceptable salt may be a hydrochloride, sulfate, phosphate, maleate, fumarate, citrate, mesylate, p-toluenesulfonate, tartrate, etc.

In some embodiments in the present application, R₂ is selected from C₁-C₃ alkyl, C₅-C₁₅ monoalkenyl, C₅-C₁₅ dienyl, C₅-C₁₅ trienyl, alkynyl, 5-6 membered cycloalkyl, phenyl, 5-6 membered heterocyclyl, 5-6 membered heteroaryl and sterol group; wherein Y and R₂ are directly connected, or Y and R₂ are connected to form a ring;

R₂ is unsubstituted or is substituted by one or more groups selected from C₁-C₆ alkyl, hydroxyl, halogen, trihalomethyl, carboxyl and phenyl.

Further, in some embodiments of the present application, R₂ is selected from methyl, ethyl, propyl, C₅ monoalkenyl, C₁₀ dienyl, C₁₅ trienyl, alkynyl, cyclopentyl, cyclohexyl, triazolyl, phenyl, piperidinyl, piperazinyl, pyrrolidinyl, pyridyl, pyrimidyl, sterol group; wherein Y and R₂ are directly connected, or Y and R₂ are connected to form a ring;
R₂ is unsubstituted or is substituted by one or more groups selected from C₁-C₆ alkyl, hydroxyl, halogen, trihalomethyl and carboxyl;
wherein the sterol group is selected from

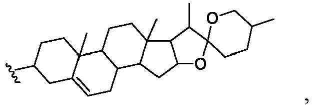

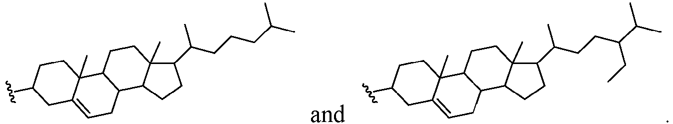

In some embodiments of the present application, R₃ is selected from hydrogen, halogen, amino, acetyl, 5-6 membered heterocyclyl, phenyl, biphenyl, naphthyl, 5-6 membered heteroaryl, 5-6 membered cycloalkyl, ester group, carboxyl, amido, trihalomethyl and adamantyl;
R₃ is unsubstituted, or is substituted by one or more groups selected from C₁-C₆ alkyl, hydroxyl, halogen, trihalomethyl, carboxyl and phenyl.

Further, in some embodiments of the present application, R₃ is selected from hydrogen, halogen, amino, hydroxyl, acetyl, phenyl, biphenyl, naphthyl, cyclopentyl, cyclohexyl, piperidinyl, piperazinyl, pyrrolidinyl, pyridyl, pyrimidyl, ester group, carboxyl, amido, trihalomethyl and adamantyl;
R₃ is unsubstituted or is substituted by one or more groups selected from C₁-C₆ alkyl, hydroxyl, halogen, trihalomethyl, carboxyl and phenyl.

In some embodiments of the present application, the compound has a structure of Formula I: 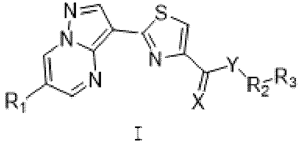 wherein X, Y, R₁, R₂ and R₃ are as defined above.

In these embodiments, in the compound of Formula I, X is O or S; Y is -O-, -NH- or 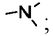
R₁ is hydrogen or C₁-C₂ alkyl;
R₂ is selected from methyl, ethyl, C₅ monoalkenyl, C₁₀ dienyl, cyclohexyl, phenyl and pyridyl; wherein Y and R₂ are directly connected, or Y and R₂ are connected to form a ring;
R₃ is selected from hydrogen, phenyl, pyridyl, pyrimidyl, ester group, trihalomethyl;
R₂ or R₃ is unsubstituted, or is substituted by one or more groups selected from C₁-C₆ alkyl, hydroxyl, halogen, trihalomethyl and carboxyl.

In these embodiments, in the compound of Formula II, X is O or S; Y is -O-, -NH- or 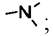
R₂ is selected from methyl, ethyl, propyl, C₅ monoalkenyl, C₁₀ dienyl, C₁₅ trienyl, alkynyl, cyclopentyl, cyclohexyl, phenyl, triazolyl, pyridyl and sterol group; wherein Y and R₂ are directly connected, or Y and R₂ are connected to form a ring;
the sterol group is selected from 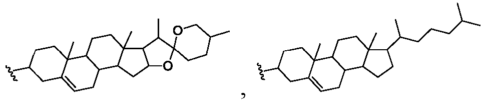 and 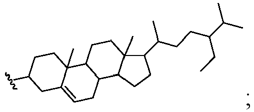
R₃ is selected from hydrogen, halogen, amino, hydroxyl, acetyl, phenyl, biphenyl, naphthyl, cyclopentyl, cyclohexyl, pyrrolidinyl, pyridyl, pyrimidyl, ester group, carboxyl, amido, trihalomethyl and adamantyl;
R₂ or R₃ is unsubstituted or is substituted by one or more groups selected from C₁-C₆ alkyl, hydroxyl, halogen, trihalomethyl, carboxyl and phenyl.

In some embodiments of the present application, when X is O, the compound has a structure of Formula IA: 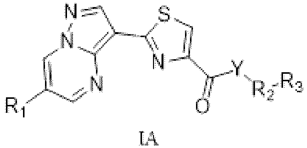 wherein Y, R₁, R₂ and R₃ are as defined above; Y and R₂ are directly connected, or Y and R₂ are connected to form a ring.

In some embodiments of the present application, when X is S, the compound has a structure of Formula IB: 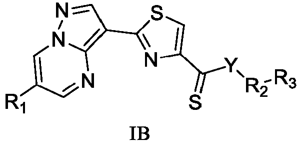
wherein, Y is -NH-, R₁, R₂ and R₃ are as defined above;
further, in some embodiments, in the compound of Formula IB, R₂ is selected from methyl, ethyl and pyridyl; R₃ is selected from hydrogen, pyridyl and pyrimidyl.

In some embodiments of the present application, when Y is -O-, -NH- or 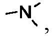 X is O;
wherein, when Y is -O- or -NH-, Y and R₂ are directly connected;
or, when Y is 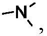 Y and R₂ are connected to born a cyclic R₂' structure, and the N atom is a ring-forming atom on the R₂' structure;
preferably, the compound has a structure of Formula IA₁, IA₂, IA₃: 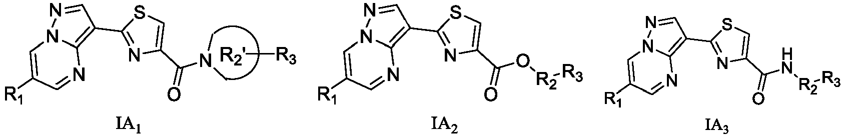
wherein, R₁, R₂ and R₃ are as defined above; R₂' is selected from 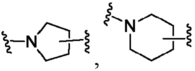 and

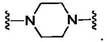

In some embodiments of the present application, R₂' is selected from 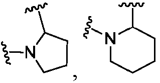 and

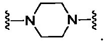

Further, in some embodiments of the present application, in Formula IIA₂, R₂ is selected from 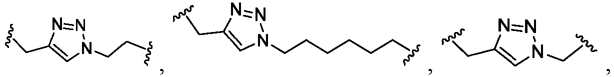 and R₃ is selected from halogen, hydroxyl, phenyl, naphthyl and adamantyl.

As examples, the present application provides a series of compounds selected from the following structures:

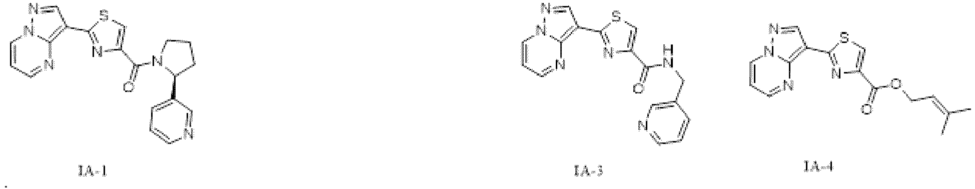

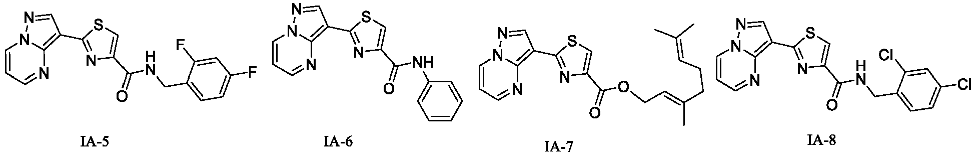

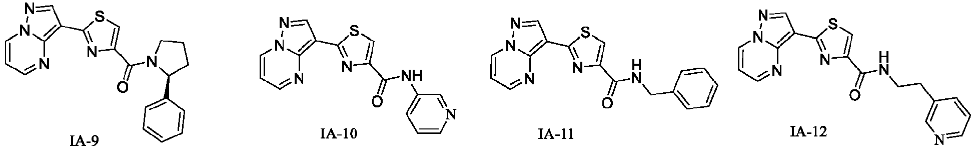

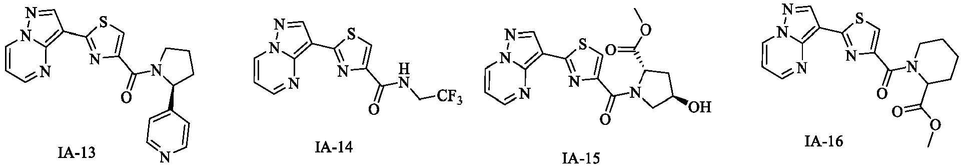

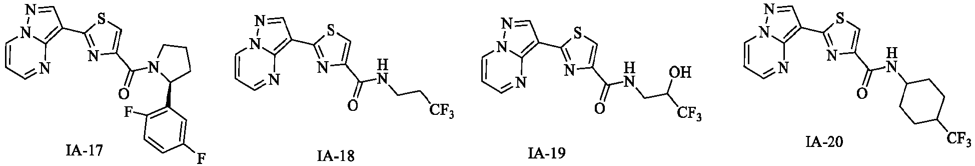

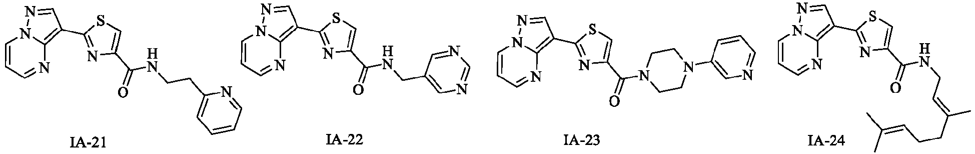

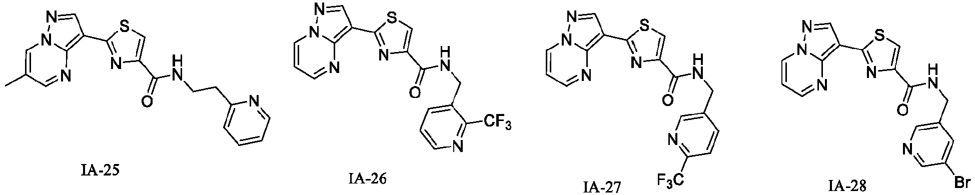

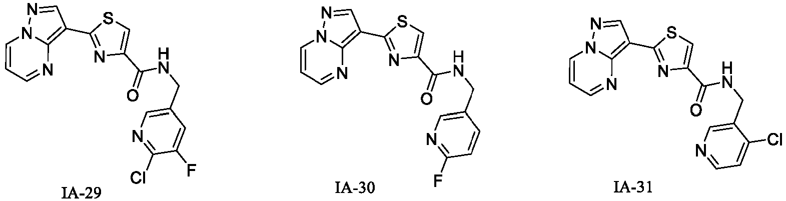

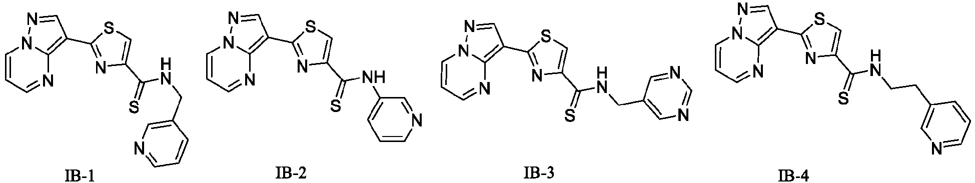

In a second aspect of the present application, the present application provides a method for preparing the compounds containing a 2,4-thiazole ring or pharmaceutically acceptable salts thereof described in the first aspect above, the method comprises:
cyclizing compound 1 with a compound 2 to obtain a compound 3; hydrolyzing the ester bond of the compound 3 to obtain a compound 4; acyl-chlorinating and aminating the compound 4 to obtain a compound 5; substituting the compound 5 with sulfur to obtain a compound 6; cyclizing the compound 6 to obtain a compound 8; hydrolyzing the ester bond of the compound 8 to obtain a compound 9; performing amide condensation or ester condensation between the compound 9 and a compound 10 to obtain a compound of Formula IA;
wherein compounds 1-6 and 8-10 are as follows: 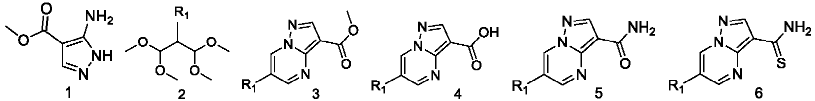 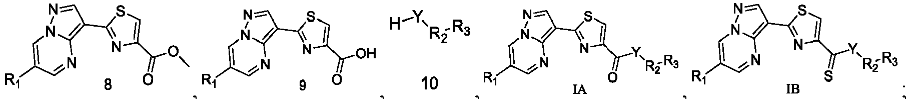 R₁, R₂, R₃ and Y are as defined above in the first aspect.

Technicians in the art can experimentally select suitable reaction conditions according to the preparation method disclosed in the present application, including but not limited to selecting a reaction solvent, selecting reaction temperature, and deciding whether to add a catalyst, etc.

In a third aspect of the present application, the present application provides a pharmaceutical composition or pharmaceutical formulation, comprising the compound or a pharmaceutically acceptable salt thereof described in the first aspect above.

Alternatively, the pharmaceutical composition or pharmaceutical formulation further comprises a pharmaceutically acceptable excipient or pharmaceutical carrier.

The pharmaceutically acceptable excipient refers to an inert or inactive substance that may be used in the production of a drug or pharmaceutical, which is non-toxic to the subject. The pharmaceutically acceptable excipients include but are not limited to solvents, co-solvents, fillers, lubricants, disintegrants, buffers, stabilizers and preservatives, etc.

The pharmaceutical carrier may be a pharmaceutically acceptable solvent, a suspending agent or a carrier for delivering a compound into an animal or a human body. The carrier may be liquid or solid and is selected according to the planned administration mode. Proteins and liposomes may also be pharmaceutical carriers.

Technicians in the art may use techniques well known in the art to formulate compounds of the present application into pharmaceutical compositions or pharmaceutical formulations. For example, the preparation of pharmaceutical formulations can be carried out according to the guidance of the Modern Pharmaceutical Preparation Series edited by Shenyang Pharmaceutical University. Suitable pharmaceutical excipients, except as mentioned herein, are known in the art, for example, see Handbook of Pharmaceutical Excipients (the fourth edition), authors are Raymond C Rowe and Paul J Sheskey.

In a fourth aspect of the present application, the present application provides the compounds or pharmaceutically acceptable salts thereof described in the first aspect above, or pharmaceutical compositions or pharmaceutical formulations described in the third aspect above, for use in the prevention and/or treatment of diseases or conditions related to the anti-activation of the immune system.

Alternatively, the present application provides the compounds or pharmaceutically acceptable salts thereof described in the above first aspect, or the pharmaceutical compositions or pharmaceutical formulations described in the third aspect above, for use as immunosuppressive drugs.

In embodiments of the present application, the disease or condition is selected from the group consisting of rejection of organ, tissue or cell transplantation, graft-versus-host disease caused by transplantation, autoimmune syndrome, and diseases or conditions associated with cytokine storm. preferably, the autoimmune syndrome includes lupus, systemic lupus erythematosus, psoriasis, eczema, dermatitis, arthritis, rheumatoid arthritis, spinal arthritis, gouty arthritis or other arthritic conditions, multiple sclerosis, dermatomycosis, antiphospholipid antibody syndrome, struma lymphomatosa, lymphocytic thyroiditis, multiple sclerosis, myasthenia gravis, type 1 diabetes, mellitus, uveitis, episcleritis, scleritis, Kawasaki's disease, uveoretinitis, choroiditis, uveitis associated with Behcet's syndrome, uveoencephalitis, viral encephalomyelitis, chronic allograft vascularopathy, post-infectious autoimmune diseases, rheumatic fever and post-infectious glomerulonephritis, inflammatory and cytoplastic dermatosis, psoriasis, psoriatic arthritis, atopic dermatitis, myopathy, myositis, osteomyelitis, contact dermatitis, dermatitis eczematosa, seborrheic dermatitis, lichen planus, pemphigus, urticaria, angioedema, angiitis, rubeola, acne vulgaris and mast-cell disease;

Further, the disease or condition associated with cytokine storm is cytokine storm syndrome caused by infectious diseases, including but not limited to tumors, inflammation, cytokine storm syndrome caused by infectious diseases such as COVID-19, etc.

In a fifth aspect of the present application, the present application provides a method for preventing and/or treating a disease or condition associated with activating immune system, comprising administering to a subject a therapeutically effective amount of a compound or a pharmaceutically acceptable salt thereof described in the first aspect above, or a pharmaceutical composition or pharmaceutical formulation described in the third aspect above.

Wherein the subject refers to an animal in need of treatment, observation or experiment, or an animal in treatment, observation or experiment, or an animal that has been subjected to treatment, observation or experiments; the animal particularly refers to a mammal, in particular a human, bovine, rat and mouse.

The therapeutically effective amount refers to an amount of a compound or a pharmaceutically acceptable salt thereof described in the first aspect above, or an amount of a pharmaceutical composition or pharmaceutical formulation comprising the compound or a pharmaceutically acceptable salt thereof, which may cause a biological or medical response of a tissue system, an animal or a person pursued by researchers, veterinarians, doctors, or other medical personnel, including alleviating or partially alleviating symptoms of the treated disease, syndrome, condition or disorder.

Compared with the existing technology, the present application has the following advantages:
The present application provides a series of novel compounds containing a 2,4-thiazole ring, which are synthesized by a simple and efficient method with high yields and low toxicity. These compounds have significant inhibitory effects on T lymphocytes and B lymphocytes and can be used for the preparation of immunosuppressive drugs.

### BRIEF DESCRIPTION OF THE DRAWINGS

The accompanying drawings to the specification, which form part of the present application, are used to provide a further understanding of the present application, and the illustrative embodiments of the present application and the description thereof are used to explain the present application. Hereinafter, embodiments of the present application are described in detail with reference to the accompanying drawings, wherein:
Fig. 1 shows IC₅₀ curves for some representative compounds of the present application.
Fig. 2 shows the effect of some representative compounds of the present application on lymphocyte viability at concentrations of 5, 2.5, 1.25, and 0.625 µM.
Fig. 3 shows the concentrations of IL-6, IL-2, TNF-a, and IFN-y in the sera of mice from different groups in Example 6.
Fig. 4 shows the H&E staining of lung tissues from mice in different groups in Example 6.
Fig. 5 shows the concentrations of IL-6, IL-2, and TNF-a in the sera of mice from different groups in Example 7.

### DESCRIPTION OF THE PREFERRED EMBODIMENTS

The present application is further described below with reference to specific embodiments.

In the examples,¹H NMR and ¹³C NMR were recorded by Avance III-400 or Avance III-600 NMR instrument with chemical shifts expressed as δ (ppm); mass spectrometry was recorded by MS-LCQ-DECA ion trap mass spectrometer (ESI/LR) and MS-Q-TOF quadrupole-time-of-flight mass spectrometry (ESI-HR); 200-300 mesh silica gel (Sinopharm Chemical Reagent Co., Ltd.) was used for compound separation.

The present invention provides a series of compounds containing a 2,4-thiazole ring, and the compounds can be prepared by the following method:

### Preparation route I:

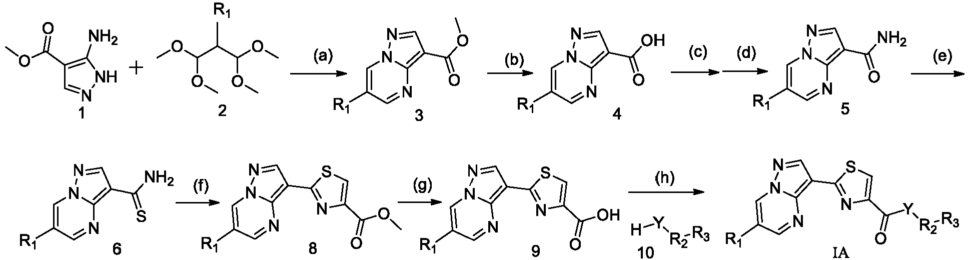

wherein R₁, R₂, R₃ and Y are as defined in the summary of the present application.

The reaction step comprises:
(a) cyclizing compound 1 with a compound 2 to obtain a compound 3; the cyclization reaction is carried out in an organic solvent, under acidic conditions, the organic solvent is an organic acid.
(b) hydrolyzing the ester bond of the compound 3 to obtain a compound 4; the ester bond hydrolysis reaction is carried out in the presence of an organic solvent, under basic conditions or acidic conditions. The organic solvent is methanol or tetrahydrofuran.
(c, d) acyl-chlorinating (c) and aminating (d) the compound 4 to obtain a compound 5; in step (c), the acyl-chlorination is carried out in the presence of an organic solvent and an acyl-chlorination reagent; the organic solvent is dichloromethane, dichloroethane, toluene or acyl chlorination reagent; the acyl-chlorination reagent is thionyl chloride, phosphorus trichloride, phosgene or oxalyl chloride; in step (d), the amination is carried out in ammonia water.
(e) substituting the compound 5 with sulfur to obtain a compound 6; the thio-substitution reaction is carried out in an organic solvent; the organic solvent is toluene; the thio-substitution reagent is Lawesson's reagent.
(f) cyclizing the compound 6 to obtain a compound 8; the cyclization reaction is carried out in an organic solvent; the organic solvent is ethanol.
(g) hydrolyzing the ester bond of the compound 8 to obtain a compound 9; the ester bond hydrolysis reaction is similar to step (b).
(h) performing amide condensation or ester condensation between the compound 9 and a compound 10 to obtain a compound of Formula IA; the amide condensation reaction is catalyzed by a condensing agent and a base in an organic solvent; the organic solvent is dichloromethane or N,N-dimethylformamide (DMF); The condensing agent is 2-(7-azabenzotriazole)-tetramethyluronium hexafluorophosphate (HATU) or dicyclohexylcarbodiimide (DCC); the base is *N,N-*diisopropylethylamine (DIPEA) or 4-dimethylaminopyridine (DMAP).

Wherein preparing a compound of Formula IB by oxidation sulfur exchange of a compound of formula IA, which reacts as follows:

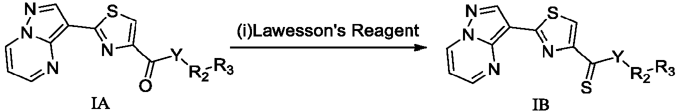

### Preparation route II:

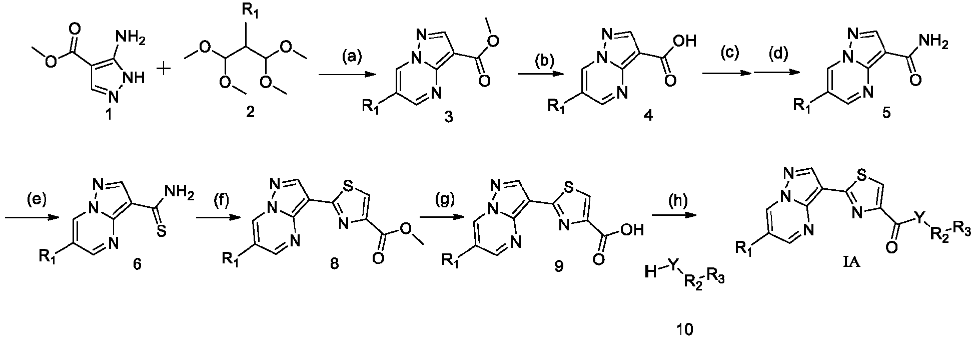

wherein R₁, R₂, R₃ and Y are as defined in the summary of the present application.
(j) reacting compound 11 with oxalyl chloride to obtain compound 12; the reaction is carried out in an organic solvent; the organic solvent is ethyl ether.
(k) aminating compound 12 to obtain compound 13; the amination is similar to step (d).
(l) oxidizing compound 13 to obtain compound 14; the oxidation reaction is carried out under conditions of organic solvent and oxidant; the organic solvent is N,N-dimethylformamide (DMF); the oxidant is dichlorosulfoxide.
(m) cyclizing compound 14 to obtain compound 16; the cyclization reaction is carried out under conditions of organic solvent and basic catalyst; the organic solvent is *N,N-*dimethylformamide; the basic catalyst is 1,8-diazabicyclo[5.4.0]undec-7-ene (DBU).
(n) oxidizing compound 16 to obtain compound 17; the oxidation reaction is carried out under conditions of organic solvent and oxidant; the organic solvent is tetrahydrofuran; the oxidant is manganese dioxide.
(o) hydrolyzing the ester bond of compound 17 to obtain compound 18; the ester bond hydrolysis is similar to step (b).
(p) condensing compound 18 with a compound 19 to obtain a compound of Formula IIA; the amide condensation reaction is similar to step (h).

Specifically, the preparation method and activity of exemplary compounds are illustrated in the following examples. Those skilled in the art can prepare more compounds that conform to the generic structure of the present application based on the disclosure of the present application.

### Example 1 Compound IA-1

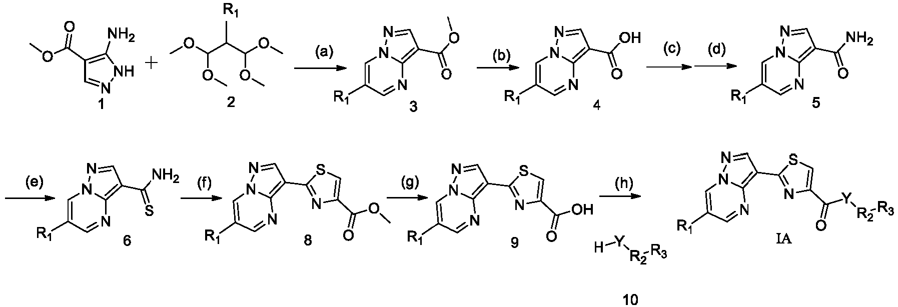

(A) Compound 1 (methyl 5-amino-1H-pyrazole-4-carboxylate) was dissolved in acetic acid, and then compound 2 (1,1,3,3-tetramethoxypropane) was added. The mixture was heated with stirring until the reaction was complete, then cooled to room temperature. The solvent was evaporated under reduced pressure, and the residue was extracted and washed with ethyl acetate and saturated sodium bicarbonate solution three times. After washing the organic phase with saturated brine once, the mixture was dried over anhydrous sodium sulfate, filtered, concentrated, and purified by column chromatography to obtain compound 3.
(b) Compound 3 was dissolved in methanol, and then a solution of sodium hydroxide was added. The reaction mixture was stirred until complete reaction, then acidified with hydrochloric acid, filtered, and dried to obtain compound 4.
(c) Compound 4 was added into dichloromethane to form a suspension, and then added with thionyl chloride and catalytic amount of DMF, the mixture was heated until the reaction was complete, then evaporated the solvent under reduced pressure to obtain an acyl chlorination product.
(d) Potassium hydroxide and ammonium chloride were dissolved in water, and then the compound obtained in step (c) was added, the mixture was stirred until the reaction was complete, filtered, and the filtrate was washed with water, and then dried to obtain compound 5.
(e) Compound 5 was dissolved in anhydrous toluene, and the Lawesson's reagent was added, the mixture was heated until the reaction was complete, then cooled to room temperature. After filtration, the filter cake was washed with toluene and then dried to yield compound 6.
(f) Compound 6 was dissolved in ethanol, and then methyl bromopyruvate was added. The resulting mixture was heated to complete reaction, then cooled to room temperature. The solvent was evaporated under reduced pressure, and the residue was extracted with water and ethyl acetate three times, and the organic layers were combined. After washing once with saturated brine, the mixture was dried over anhydrous sodium sulfate, filtered, and concentrated. The resulting material was purified by column chromatography to obtain compound 8.
(g) Compound 8 was dissolved in methanol, and aqueous sodium hydroxide solution was added, the mixture was stirred until the reaction was complete, and then acidified with hydrochloric acid, filtered, dried to obtain compound 9.
(h) Compound 9 was dissolved in dichloromethane, HATU was added, and DIEA was dropwise added at a low temperature, the mixture was stirred for 10 min, compound 10 was added, the mixture was stirred at room temperature for 30 min, the reaction was quenched with water, the reaction solution was extracted with dichloromethane for three times, organic phases were combined, and washed with saturated aqueous solution of sodium chloride, dried with anhydrous sodium sulfate, filtered and concentrated, and separated and purified by column chromatography to obtain compound IA.

Amines or alcohols containing different substituents were used as compound 10, and different compounds IA were prepared according to steps similar to those described previously in this example, and the structures of compound 10 and the prepared compounds IA are listed in Table 1:

**Table 1**

| No. | Compound 10 | Structural formula | No. | Compound 10 | Structural formula |
|---|---|---|---|---|---|
| IA-1 | 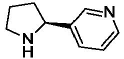 | 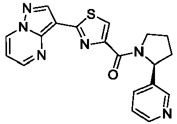 | | | |
| IA-3 | 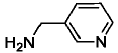 | 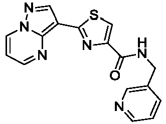 | IA-4 | 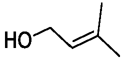 | 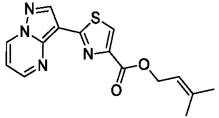 |
| IA-5 | 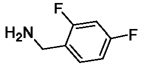 | 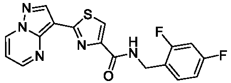 | IA-6 | 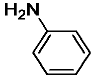 | 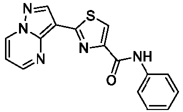 |
| IA-7 | 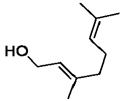 | 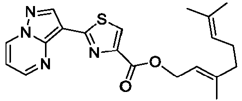 | IA-8 | 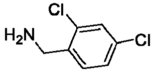 | 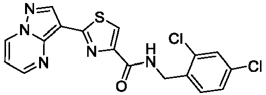 |
| IA-9 | 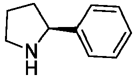 | 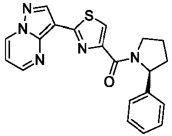 | IA-10 | 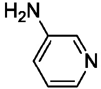 | 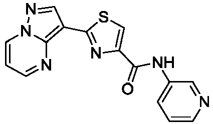 |
| IA-11 | 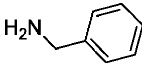 | 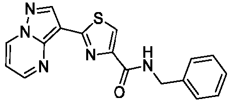 | IA-12 | 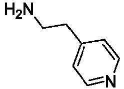 | 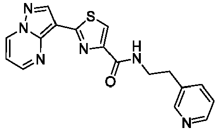 |
| IA-13 | 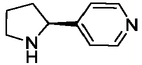 | 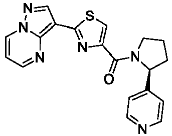 | IA-14 | 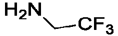 | 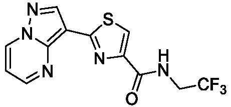 |
| IA-15 | 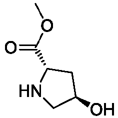 | 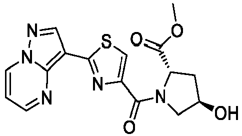 | IA-16 | 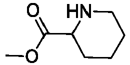 | 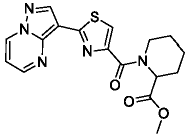 |
| IA-17 | 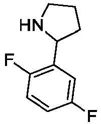 | 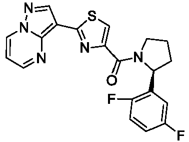 | IA-18 | 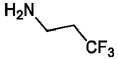 | 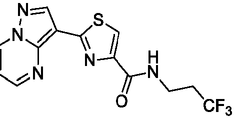 |
| IA-19 | 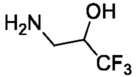 | 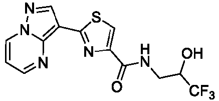 | IA-20 | 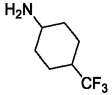 | 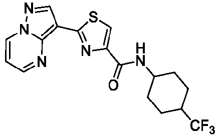 |
| IA-21 | 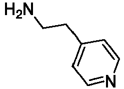 | 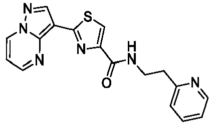 | IA-22 | 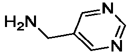 | 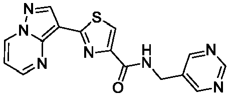 |
| IA-23 | 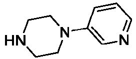 | 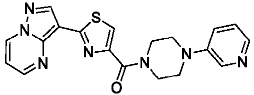 | IA-24 | 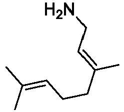 | 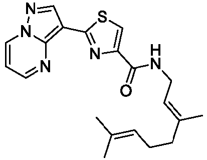 |
| IA-25 | 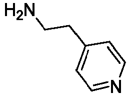 | 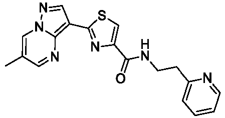 | IA-26 | 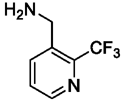 | 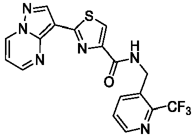 |
| IA-27 | 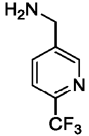 | 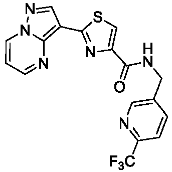 | IA-28 | 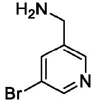 | 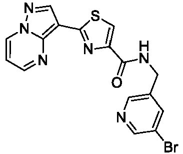 |
| IA-29 | 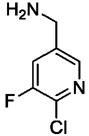 | 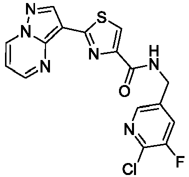 | IA-30 | 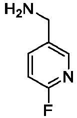 | 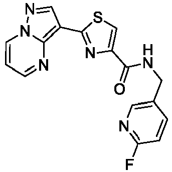 |
| IA-31 | 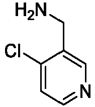 | 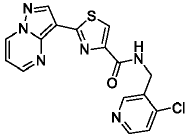 | | | |

Compound IA-1: yellow solid, the yield was 87.2%. ¹H NMR (400 MHz, CDCl₃ ) δ 9.29 - 9.21 (m, 2H), 8.83 (dd, *J =* 15.0, 3.1 Hz, 1H), 8.64 - 8.60 (m, 1H), 8.47 - 8.39 (m, 1H), 7.88 (s, 1H), 7.65 (dt, *J =* 15.0, 3.1 Hz, 1H), 7.28 (td, *J =* 14.9, 4.7 Hz, 2H), 4.74 - 4.66 (m, 1H), 4.04 (dt, *J =* 19.8, 13.3 Hz, 1H), 3.81 (dt, *J =* 24.8, 13.2 Hz, 1H), 2.24 (dtd, *J =* 12.0, 8.8, 1.6 Hz, 1H), 2.13 - 1.88 (m, 3H).

Compound IA-3: yellow solid, the yield was 86.5%. ¹H NMR (400 MHz, CDCl₃) δ 8.76 (d, *J =* 7.0 Hz, 1H), 8.71 (dd, *J =* 5.5, 3.5 Hz, 2H), 8.67 (s, 1H), 8.56 (dd, 1H), 8.16 (s, 1H), 7.93 (s, 1H), 7.81 (d, *J =* 7.8 Hz, 1H), 7.33(t, 1H), 7.01 (t, 1H), 4.72 (d, *J =* 5.9 Hz, 2H), 2.46 (s, *J =* 1.2 Hz, 1H).

Compound IA-4: yellow solid, the yield was 89.8%. ¹H NMR (400 MHz, DMSO-*d*₆) δ 9.38 (dd, *J =* 7.1, 1.7 Hz, 1H), 8.92 (dd, *J =* 4.1, 1.7 Hz, 1H), 8.88 (s, 1H), 8.55 (s, 1H), 7.35 (dd, *J =* 7.0, 4.2 Hz, 1H), 5.51 (td, 1H), 4.88 (d, *J =* 7.3 Hz, 2H), 1.83 (dd, *J =* 3.0, 1.3 Hz, 6H).

Compound IA-5: yellow solid, the yield was 82.3%. ¹H NMR (400 MHz, Chloroform-d) δ 8.75 (d, *J =* 7.0 Hz, 2H), 8.72 - 8.68 (m, 1H), 8.14 (s, 1H), 7.87 (t, *J =* 6.4 Hz, 1H), 7.45 (m, *J =* 8.0 Hz, 1H), 6.99 (dd, *J* = 7.1, 4.2 Hz, 1H), 6.84 (m, *J =* 9.6, 9.0 Hz, 2H), 4.69 (d, *J* = 6.3 Hz, 2H).

Compound IA-6: yellow solid, the yield was 85.5%. ¹H NMR (400 MHz, CDCl₃) δ 8.89 (s, 1H), 8.74 (dd, *J =* 7.0, 1.8 Hz, 1H), 8.67 (dd, *J =* 4.1, 1.7 Hz, 1H), 8.17 (s, 1H), 6.96 (dd, *J =* 7.0, 4.1 Hz, 1H), 5.49 (t, *J* = 6.7 Hz, 1H), 5.08 (t, *J =* 6.1 Hz, 1H), 4.90 (d, *J =* 7.1 Hz, 2H), 2.08 (td, *J =* 9.8, 7.5, 4.3 Hz, 5H), 1.76 (s, 3H), 1.66 (s, 3H), 1.58 (s, 3H).

Compound IA-7: yellow solid, the yield was 85.4%. ¹H NMR (400 MHz, CDCl₃) δ 9.25 (m,2H), 8.83 (dd, *J* = 15.0, 3.1 Hz, 1H), 7.88 (s, 1H), 7.28 (t, *J* = 15.0 Hz, 1H), 5.34(dddt, *J* = 14.3,10.3, 4.0,2.0 Hz, 1H), 5.15 (dddt, *J =* 14.3, 10.3, 4.0, 2.0 Hz, 1H), 4.67 (d, *J =* 12.4 Hz, 2H), 2.02 (m, 4H), 1.82 (d, *J =* 2.0 Hz, 3H), 1.70 (d, *J =* 2.0 Hz, 3H), 1.66 (d, *J =* 2.0 Hz, 3H).

Compound IA-8: yellow solid, the yield was 79.4%. ¹H NMR (400 MHz, CDCl₃) δ 9.24 (m, 2H), 8.83 (dd, *J =* 7.5, 1.4 Hz, 1H), 7.89 (d, *J =* 12.1 Hz, 2H), 7.75 (d, *J =* 1.4 Hz, 1H), 7.25 (m, 3H), 4.23 (s, 2H).

Compound IA-9: yellow solid, the yield was 87.3%. ¹H NMR (400 MHz, CDCl₃) δ 8.82 (s, 1H), 8.77 (dd, *J =* 7.1, 1.8 Hz, 1H), 8.71 (dd, *J =* 6.8, 1.9 Hz, 3H), 8.64 (dd, *J =* 4.1, 1.7 Hz, 2H), 8.29 (s, 2H), 8.09 (s, 1H), 7.84 (s, 2H), 7.37 - 7.25 (m, 10H), 7.23 - 7.15 (m, 7H), 6.99 (dd, *J =* 7.0, 4.1 Hz, 1H), 6.94 (dd, *J =* 7.0, 4.1 Hz, 2H), 6.01 (dd, *J =* 7.7, 2.2 Hz, 2H), 5.44 (dd, *J =* 7.8, 4.4 Hz, 1H), 4.44 (dt, *J =* 12.2, 6.6 Hz, 1H), 4.29 (dt, *J =* 11.6, 6.9 Hz, 1H), 3.98 (dd, *J =* 8.6, 5.6 Hz, 4H), 2.42 (ddt, *J =* 14.6, 7.4, 3.4 Hz, 4H), 2.16 - 1.86 (m, 11H).

Compound IA-10: yellow solid, the yield was 89.5%. ¹H NMR (400 MHz, DMSO-*d*₆) δ 9.37 (dd, *J =* 7.0, 1.6 Hz, 1H), 9.11 (d, *J =* 2.3 Hz, 1H), 9.02 (s, 1H), 8.91 (dd, *J* = 4.1, 1.6 Hz, 1H), 8.49 (s, 1H), 8.45 - 8.40 (m, 1H), 8.37 (dd, *J =* 8.3, 2.1 Hz, 1H), 7.52 (dd, *J =* 8.3, 4.7 Hz, 1H), 7.34 (dd, *J =* 7.0, 4.1 Hz, 1H).

Compound IA-11: yellow solid, the yield was 82.6%. ¹H NMR (400 MHz, CDCl₃) δ 8.66 (d, *J* = 5.2 Hz, 2H), 8.64 - 8.59 (m, 1H), 8.08 (s, 1H), 7.82 (s, 1H), 7.33 (d, *J =* 7.5 Hz, 2H), 7.28 (t, *J =* 7.2 Hz, 2H), 7.24 - 7.18 (m, 1H), 6.94 - 6.87 (m, 1H), 4.62 (d, *J =* 5.9 Hz, 2H).

Compound IA-12: yellow solid, the yield was 84.9%. ¹H NMR (400 MHz, Chloroform-d) δ 8.72 (d, *J* = 7.0 Hz, 1H), 8.65 (s, 2H), 8.52 (s, 1H), 8.47 (d, *J =* 4.1 Hz, 1H), 8.06 (s, 1H), 7.61 (d, *J =* 7.5 Hz, 2H), 7.24 (d, *J =* 6.7 Hz, 1H), 6.98 - 6.92 (m, 1H), 3.71 (q, *J =* 6.8 Hz, 2H), 2.97 (t, *J* = 7.1 Hz, 2H).

Compound IA-13: yellow solid, the yield was 87.9%. ¹H NMR (400 MHz, Chloroform-d) δ 8.74 - 8.68 (m, 2H), 8.63 (dt, *J =* 6.0, 1.7 Hz, 2H), 8.55 (dd, *J =* 4.0, 1.8 Hz, 1H), 8.51 - 8.46 (m, 2H), 8.45 - 8.39 (m, 2H), 8.09 (s, 1H), 8.02 (s, 1H), 7.94 (s, 1H), 7.21 (d, *J =* 5.4 Hz, 2H), 7.13 - 7.07 (m, 2H), 6.91 (dd, *J =* 7.0, 4.1 Hz, 1H), 6.86 (dd, *J =* 7.0, 4.0 Hz, 1H), 6.07 (dd, *J =* 7.9, 2.1 Hz, 1H), 5.29 (dd, *J =* 8.1, 5.0 Hz, 1H), 4.38 (dt, *J =* 11.7, 6.7 Hz, 1H), 4.24 (dt, *J =* 11.6, 7.0 Hz, 1H), 3.96 - 3.85 (m, 2H), 2.39 (tdt, *J =* 15.0, 12.6, 7.2 Hz, 2H), 1.96 (dtt, *J =* 12.6, 9.9, 7.6, 3.6 Hz, 5H), 1.80 (dtd, *J =* 22.6, 11.7, 11.3, 8.0 Hz, 2H).

Compound IA-14: yellow solid, the yield was 81.2%. ¹H NMR (400 MHz, CDCl₃) δ 8.76 (dd, *J* = 7.1, 1.7 Hz, 1H), 8.66 (d, *J* = 4.0 Hz, 2H), 8.12 (s, 1H), 7.00 (dd, *J* = 7.0, 4.1 Hz, 1H), 4.07 (q*, J* = 9.1 Hz, 2H).

Compound IA-15: yellow solid, the yield was 86.3%. ¹H NMR (400 MHz, DMSO-*d*₆) δ 9.29 - 9.25 (m, 2H), 8.88 - 8.80 (m, 3H), 8.66 (s, 1H), 8.26 (s, 1H), 8.23 (s, 1H), 7.26 - 7.21(m, 2H), 5.41 (t*, J* = 7.9 Hz, 1H), 5.18 (d, *J* = 3.8 Hz, 1H), 5.13 (d, *J* = 3.4 Hz, 1H), 4.58 (t, *J* = 8.4 Hz, 1H), 4.41 (s, 1H), 4.32 (q, *J* = 3.8 Hz, 1H), 4.17 - 3.99 (m, 3H), 3.74 (dt, *J* = 12.3, 2.1 Hz, 1H), 3.69 - 3.59 (m, 5H), 3.51 (s, 3H), 2.37 (td, *J* = 10.7, 8.7, 3.2 Hz, 1H), 2.26 - 2.07 (m, 2H), 1.96 (ddd, *J =* 13.2, 9.2, 4.4 Hz, 1H).

Compound IA-16: yellow solid, the yield was 86.4%. ¹H NMR (400 MHz, CDCl₃) δ 9.29 - 9.21 (m, 2H), 8.83 (dd, *J =* 15.0, 3.1 Hz, 1H), 7.88 (s, 1H), 7.28 (t, *J =* 15.0 Hz, 1H), 4.87 (t, *J =* 6.2 Hz, 1H), 3.68 - 3.42 (m, 5H), 2.35 - 2.17 (m, 1H), 2.09 - 1.90 (m, 1H), 1.87 - 1.52 (m, 4H).

Compound IA-17: yellow solid, the yield was 83.5%. ¹H NMR (400 MHz, CDCl₃ ) δ 9.28 - 9.21 (m, 2H), 8.83 (dd, *J =* 7.5, 1.4 Hz, 1H), 7.88 (s, 1H), 7.28 (t, *J =* 7.5 Hz, 1H), 7.24 - 7.06 (m, 3H), 4.59 (t, *J =* 4.6 Hz, 1H), 4.06 (dt, *J =* 13.0, 6.6 Hz, 1H), 3.88 - 3.79 (m, 1H), 2.30 - 2.15 (m, 3H), 2.07 - 1.97 (m, 1H).

Compound IA-18: yellow solid, the yield was 82.3%.¹H NMR (400 MHz, CDCl₃) δ 8.87 (s, 1H), 8.78 (d, *J* = 3.2 Hz, 1H), 8.45 (dd, *J* = 6.2, 2.6 Hz, 1H), 8.33 (s, 1H), 7.28 (t, *J* = 15.0 Hz, 1H), 3.74 (dd, *J =* 12.6, 6.3 Hz, 2H), 2.52 - 2.36 (m, 2H).

Compound IA-19: yellow solid, the yield was 89.3%.¹H NMR (400 MHz, MeOD) δ 9.06 (dd, *J* = 7.0, 1.4 Hz, 1H), 8.81 (s, 1H), 8.78 (dd, *J =* 4.0, 1.4 Hz, 1H), 8.19 (s, 1H), 7.19 (dd, *J =* 7.0, 4.1 Hz, 1H), 4.35 - 4.23 (m, 1H), 3.86 (dd, *J =* 13.9, 3.9 Hz, 1H), 3.56 (dd, *J =* 13.9, 8.5 Hz, 1H).

Compound IA-20: yellow solid, the yield was 85.7%.¹H NMR (400 MHz, CDCl₃) δ 8.81 - 8.73 (m, 2H), 8.70 (dd, *J =* 4.0, 1.5 Hz, 1H), 8.10 (s, 1H), 7.40 (d, *J =* 8.1 Hz, 1H), 6.99 (dd, *J =* 7.0, 4.1 Hz, 1H), 3.96 (tdt, *J =* 11.9, 8.2, 3.9 Hz, 1H), 2.23 (d, *J =* 10.3 Hz, 2H), 2.03 (m, *J =* 10.0 Hz, 4H), 1.51 (dt, *J =* 14.4, 12.3 Hz, 2H), 1.42 - 1.28 (m, 2H).

Compound IA-21: yellow solid, the yield was 83.4%. ¹H NMR (400 MHz, CDCl₃) δ 8.77 (dd, *J =* 7.1, 1.8 Hz, 1H), 8.70 - 8.64 (m, 2H), 8.52 (d, *J =* 4.9 Hz, 1H), 8.06 (s, 1H), 7.65 (td, *J =* 7.7, 1.8 Hz, 1H), 7.29 (d, *J =* 5.7 Hz, 1H), 7.21 - 7.15 (m, 1H), 6.99 (dd, *J =* 7.1, 4.1 Hz, 1H), 3.82 (t, *J* = 7.0 Hz, 2H), 3.34 (s, 1H), 3.12 (t, *J* = 7.0 Hz, 2H).

Compound IA-22: yellow solid, the yield was 88.6%. ¹H NMR (400 MHz, DMSO-*d*₆) δ 9.31 (dd, *J* = 7.0, 1.6 Hz, 1H), 9.15 (t, *J* = 6.1 Hz, 1H), 9.10 (s, 1H), 8.86 (dd, *J* = 4.1, 1.6 Hz, 1H), 8.81 (s, 2H), 8.27 (s, 1H), 7.29 (dd, *J* = 7.0, 4.1 Hz, 1H), 4.55 (d, *J* = 6.1 Hz, 2H).

Compound IA-23: yellow solid, the yield was 85.5%. ¹H NMR (400 MHz, CDCl₃) δ 8.77 (dt, *J =* 6.2, 3.1 Hz, 1H), 8.74 - 8.68 (m, 2H), 8.34 (d, *J =* 1.9 Hz, 1H), 8.12 (t, *J =* 4.7 Hz, 1H), 8.00 (s, 1H), 7.44 (dt, *J =* 8.6, 6.8 Hz, 2H), 7.00 (dt, *J =* 8.9, 4.5 Hz, 1H), 3.41 - 3.32 (m, 4H), 3.30 - 3.22 (m, 4H).

Compound IA-24: yellow solid, the yield was 73.5%. ¹H NMR (400 MHz, CDCl₃) δ 8.79 (s, 1H), 8.76 (dd, *J =* 7.0, 1.5 Hz, 1H), 8.71 (dd, *J =* 3.9, 1.4 Hz, 1H), 8.11 (s, 1H), 7.47 (s, 1H), 7.00 (dd, *J* = 7.0, 4.1 Hz, 1H), 5.34 (t, *J* = 6.6 Hz, 1H), 5.10 (t, *J* = 6.6 Hz, 1H), 4.10 (t, *J* = 6.2 Hz, 2H), 2.19 - 2.09 (m, 2H), 2.08 - 2.02 (m, 2H), 1.75 (s, 3H), 1.68 (s, 3H), 1.61 (s, 3H).

Compound IA-25: yellow solid, the yield was 82.2%. ¹H NMR (400 MHz, CDCl₃) δ 8.63 (s, 1H), 8.61 - 8.49 (m, 3H), 8.10 (s, 1H), 7.66 (t, *J* = 8.2 Hz, 2H), 7.31 (dd, *J* = 7.8, 4.7 Hz, 1H), 3.77 (q, *J* = 7.0 Hz, 2H), 3.02 (t, *J* = 7.2 Hz, 2H), 2.46 (s, 3H).

Compound IA-26: yellow solid, the yield was 83.5%.¹H NMR (400 MHz, CDCl₃) δ 8.78 - 8.71 (m, 2H), 8.69 (dd, *J* = 4.0, 1.7 Hz, 1H), 8.59 (d, *J* = 4.2 Hz, 1H), 8.13 (s, 1H), 8.05 (d, *J* = 7.9 Hz, 1H), 7.99 (t, *J* = 6.3 Hz, 1H), 7.47 (dd, *J* = 7.9, 4.7 Hz, 1H), 6.99 (dd, *J* = 7.0, 4.1 Hz, 1H), 4.88 (d, *J* = 6.4 Hz, 2H).

Compound IA-27: yellow solid, the yield was 79.0%. ¹H NMR (400 MHz, CDCl₃) δ 8.65 (dt, *J* = 11.8, 6.5 Hz, 4H), 8.09 (s, 1H), 8.03 (t, *J* = 6.4 Hz, N-H), 7.85 (d, *J* = 8.2 Hz, 1H), 7.56 (d, *J* = 8.0 Hz, 1H), 6.93 (dd, *J =* 7.0, 4.1 Hz, 1H), 4.68 (d, *J =* 4.4 Hz, 2H).

Compound IA-28: yellow solid, the yield was 81.3%. ¹H NMR (400 MHz, DMSO-d6) δ 9.29 (dd, *J* = 7.1, 1.7 Hz, 1H), 9.13 (t, *J* = 6.3 Hz, 1H), 8.84 (dd, *J* = 4.1, 1.7 Hz, 1H), 8.81 (s, 1H), 8.59 (dd, *J =* 8.9, 2.0 Hz, 2H), 8.26 (s, 1H), 8.02 (t, *J =* 2.1 Hz, 1H), 7.27 (dd, *J =* 7.0, 4.1 Hz, 1H), 4.54 (d, *J =* 6.2 Hz, 2H).

Compound IA-29: yellow solid, the yield was 73.3%. ¹H NMR (400 MHz, CDCl3) δ 8.79 - 8.68 (m, 3H), 8.22 (d, *J =* 1.2 Hz, 1H), 8.16 (s, 1H), 8.03 (t, *J =* 5.9 Hz, 1H), 7.56 (dd, *J =* 8.5, 1.7 Hz, 1H), 7.01 (dd, *J =* 7.0, 4.1 Hz, 1H), 4.67 (d, *J =* 6.4 Hz, 2H).

Compound IA-30: yellow solid, the yield was 83.5%.¹H NMR (400 MHz, CDCl3) δ 8.79 - 8.68 (m, 1H), 8.23 (s, 1H), 8.17 (d, *J =* 5.8 Hz, 1H), 7.94 (s, 1H), 7.87 (d, *J =* 5.4 Hz, 1H), 7.00 (dd, *J =* 7.0, 4.1 Hz, 1H), 6.93 (d, *J =* 4.1 Hz, 1H), 4.68 (d, *J =* 6.3 Hz, 1H).

Compound IA-31: yellow solid, the yield was 83.6%. ¹H NMR (400 MHz, CDCl3) δ 8.76 (d, *J =* 7.0 Hz, 1H), 8.72 (d, *J =* 4.4 Hz, 2H), 8.42 (s, 1H), 8.16 (s, 1H), 7.93 (s, 1H), 7.74 (dd, *J =* 8.1, 1.8 Hz, 1H), 7.30 (t, *J =* 7.3 Hz, 1H), 7.01 (dd, *J =* 6.9, 4.1 Hz, 1H), 4.68 (d, *J =* 6.3 Hz, 2H).

### Example 2 Compound IB-1

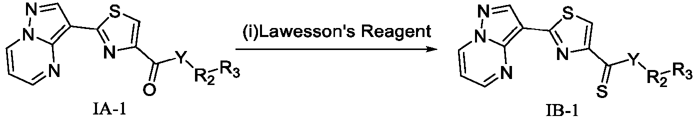

(i) Compound IA-1 was dissolved in anhydrous toluene, and Lawesson's reagent was added, the mixture was heated until the reaction was complete, then cooled to room temperature. After filtration, the filter cake was washed with toluene and then dried to yield compound IB-1.

Compound IA containing different substituents were used as starting materials, and different compounds IB were prepared according to steps similar to those described previously in this example, and the structures of compound IA and the prepared compounds IB are listed in the below Table:

| No. | Compound IA | Structural formula | No. | Compound IA | Structural formula |
|---|---|---|---|---|---|
| IB-1 | 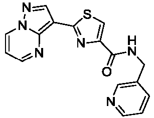 | 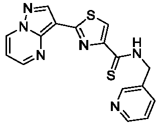 | IB-2 | 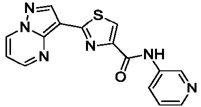 | 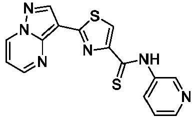 |
| IB-3 | 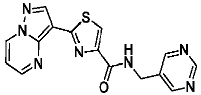 | 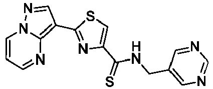 | IB-4 | 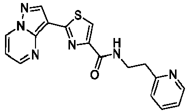 | 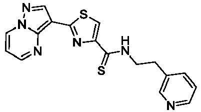 |

Compound IB-1: yellow solid, the yield was 69.2%. ¹H NMR (400 MHz, CDCl3) δ 9.25 (dd, *J* = 15.0, 3.1 Hz, 1H), 8.83 (dd, *J* = 15.0, 3.1 Hz, 1H), 8.62 (m, 1H), 8.43 (m, 2H), 7.88 (s, 1H), 7.65(dt, J=15.02,3.06 1H), 7.28 (td, *J =* 14.9, 4.7 Hz, 2H), 4.40 (s, 2H).

Compound IB-2: yellow solid, the yield was 77.3%. ¹H NMR (400 MHz, CDCl3) δ 9.25 (dd, *J* = 7.4, 1.5 Hz, 1H), 8.83 (dd, *J* = 7.5, 1.4 Hz, 1H), 8.50 (d, *J* = 1.3 Hz, 1H), 8.44 (s, 1H), 8.13 (dd, *J* = 7.5, 1.3 Hz, 1H), 7.88 (s, 1H), 7.27 (dt, *J* = 9.7, 7.5 Hz, 2H), 7.09 (dt, *J* = 7.5, 1.5 Hz, 1H).

Compound IB-3: yellow solid, the yield was 73.7%. ¹H NMR (400 MHz, CDCl3) δ 9.41 (s, 1H), 9.25 (dd, *J =* 15.0, 3.1 Hz, 1H), 8.83 (dd, *J =* 15.0, 3.1 Hz, 1H), 8.61 (s, 2H), 8.54 (s, 1H), 8.44 (s, 1H), 7.88 (s, 1H), 7.26 (d, *J* = 15.0 Hz, 1H), 4.40 (s, 2H).

Compound IB-4: yellow solid, the yield was 75.0%. ¹H NMR (400 MHz, CDCl3) δ 9.25 (dd, *J =* 7.4, 1.5 Hz, 1H), 9.00 (s, 1H), 8.83 (dd, *J =* 7.5, 1.4 Hz, 1H), 8.42(m, 3H), 7.88 (s, 1H), 7.46 (dt, *J* = 7.5, 1.4 Hz, 1H), 7.28 (t, *J* = 7.5 Hz, 1H), 7.16 (t, *J* = 7.5 Hz, 1H), 3.54 (t, *J* = 7.6 Hz, 2H), 2.81 (t, *J* = 7.6 Hz, 2H).

### Example 3 Testing of the in vitro immunosuppressive activity of the compounds

Lymphocytotoxicity test: rats were euthanized by spinal dislocation method, their spleens were aseptically removed and ground to make a single cell suspension. After removing red blood cells with 2 mL of red blood cell lysis buffer, the cell concentration was adjusted to 2×10⁶ cells/mL using RPMI-1640 medium containing 10% fetal bovine serum (FBS). Next, 100 µL of the 2×10⁶ cells/mL cell suspension and 100 µL of the appropriate concentration of the tested compound were added to each well of a 96-well plate, and the plate was incubated at 37°C with 5% CO₂ for 48 hours. At the end of the incubation period, 10 µL of CCK8 was added to each well, and the plate was returned to the incubator for 5-7 hours. The OD450 value was then measured at 450 nm using an enzyme-linked immunosorbent assay reader.

Lymphocyte proliferation assay: fresh spleen cells at a concentration of 2×10⁶ cells/mL were incubated for 48 hours in an incubator maintained at 37°C and 5% CO₂ for 48 hours, and the culture medium was induced for cell proliferation with 5 µg/mL of ConA, and the appropriate concentration of the tested compound was added to test its inhibitory activity on lymphocyte proliferation. The test results of some compounds are shown in the table below and figures accompanying the application:

| Compound No. | Inhibition rate (%) | Compound No. | Inhibition rate (%) | Compound No. | Inhibition rate (%) |
|---|---|---|---|---|---|
| IA-1 | 81.38 | IA-23 | 60.57 | | |
| | | IA-24 | 55.71 | | |
| IA-3 | 97.09 | IA-25 | 37.24 | | |
| IA-4 | 28.23 | | | | |
| IA-5 | 47.15 | | | | |
| IA-6 | 40.69 | | | | |
| IA-7 | 52.55 | | | | |
| IA-8 | 43.39 | | | | |
| IA-9 | 58.26 | | | | |
| IA-10 | 59.61 | | | | |
| IA-11 | 70.42 | | | | |
| IA-12 | 83.63 | | | | |
| IA-13 | 59.01 | | | | |
| IA-15 | 30.78 | | | | |
| IA-16 | 28.83 | | | | |
| IA-17 | 46.55 | | | | |
| IA-18 | 43.09 | | | KYN | 51.65 |
| IA-19 | 41.89 | | | ITE | 57.96 |
| IA-20 | 42.49 | | | Tacrolimus | 57.54 |
| IA-21 | 44.14 | | | | |
| IA-22 | 32.73 | | | | |

Wherein Tacrolimus is positive drug Tacrolimus; ITE is an endogenous AhR partial agonist (natural endogenous ligand of AHR); KYN (kynurenine) is metabolized by Trp via the dioxygenases TDO and IDO and is an AhR agonist.

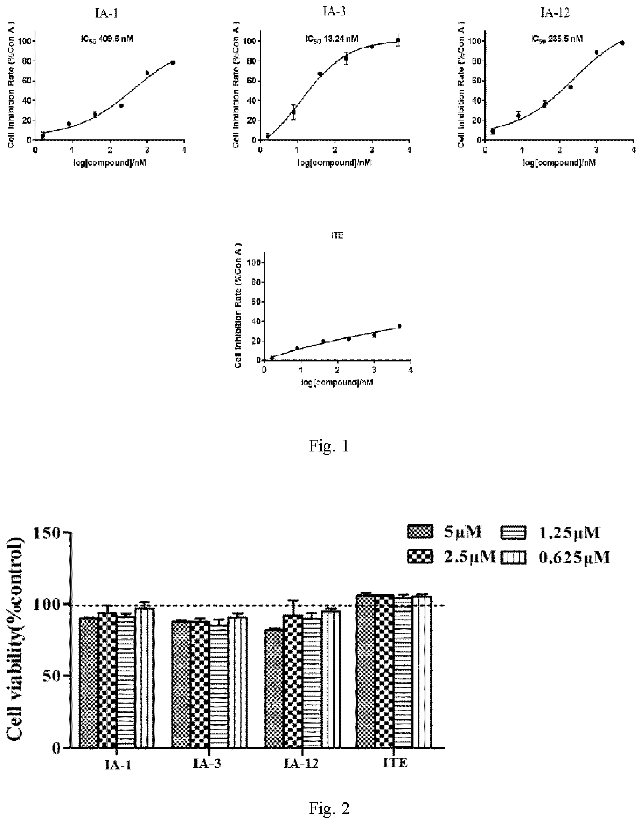

The compounds obtained in the example of the present application have better inhibitory activity on lymphocyte proliferation, wherein IC₅₀ values of some compounds are shown in the following table, wherein the IC₅₀ curves of some compounds are shown in Fig.1:

| Compounds | IC₅₀ (nM) | Compounds | IC₅₀ (nM) |
|---|---|---|---|
| | | IA-9 | 398.57±15.24 |
| | | IA-10 | 421.33±21.07 |
| | | IA-11 | 263.08±13.39 |
| | | IA-12 | 235.58±17.23 |
| | | IA-13 | 409.62±33.52 |
| IA-1 | 209.62±8.87 | IA-17 | 435.85±33.38 |
| | | IA-21 | 488.3±21.96 |
| IA-3 | 13.24±2.05 | IA-23 | 349.82±16.98 |
| IA-4 | 437.91±12.35 | IA-24 | 457.23±30.57 |
| IA-7 | 487.13±18.31 | Tacrolimus | 504.08±16.22 |

In addition, the toxicity of the compounds on lymphocytes was also tested in this example. The testing method was as follows: the cell density was 3×10⁶ cells/mL, and 100 µL of the cell suspension containing 3×10⁵ cells/well was added to each well of a 96-well plate without any stimulation factor. The toxicity of the compounds was then tested at concentrations of 5, 2.5, 1.25, and 0.625 µM. The test results showed that the compound in the present application had no obvious toxicity at the test concentration, and the results were shown in Fig. 2.

The results showed that the compounds of the present application have low toxicity to lymphocytes and have a significant inhibitory effect on lymphocytes proliferation. Among them, IA-3 has a particularly significant inhibitory effect on lymphocytes and has less cytotoxicity and high safety index. The remaining compounds also showed significant inhibitory activity on the proliferation of rat lymphocytes and were generally superior to the positive drug Tacrolimus.

In conclusion, it can be concluded from these facts that the compounds of the present application are effective in inhibiting immune cell activity, can be used for the prevention or treatment of immune diseases, and have very promising applications in the preparation of immunosuppressive drugs.

### Example 4 Experimental study on inhibition of LPS-induced cytokine storm by compounds

A total of 15 healthy male mice were randomly and equally divided into 3 groups, namely, control group, model group and drug administration group, with 5 mice in each group, labeled and their body weight recorded. Respectively administered: the control group and the model group were injected with normal saline via tail vein respectively, and the administration group was injected with the prepared in Examples 1-2 via tail vein respectively; the dose was 10 mg/kg (2 mg/mL; 50 µL/10 g). One hour later, LPS was injected intraperitoneally at 3 mg/kg (0.3 mg/mL, 100 µL/ 10g). A second injection of the drug was given 5 hours later. Blood was collected from the mice 9 hours later, followed by euthanasia.

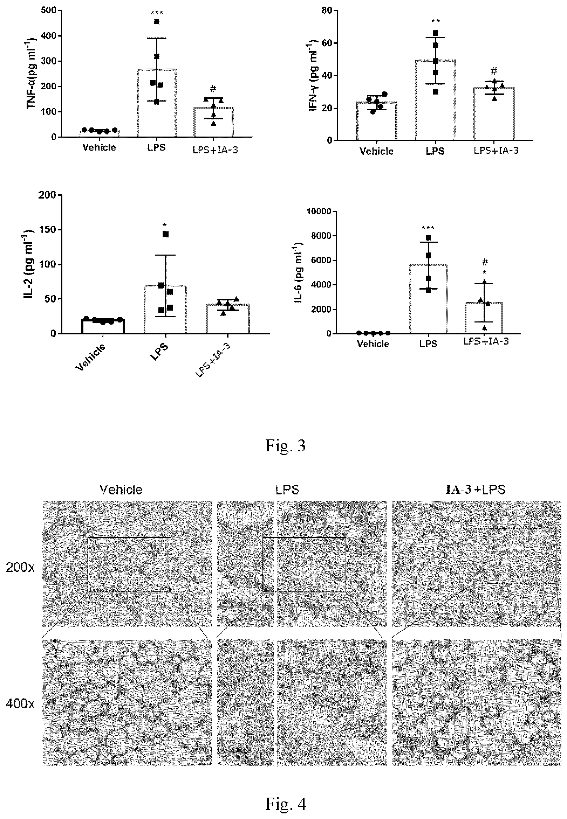

ELISA was performed to determine levels of IL-6, IL-2, TNF-a and IFN-y in serum. The results showed that the compounds of the present application exhibited different degrees of reduction in the levels of LPS-induced inflammatory factors IL-6, IL-2, TNF-a and IFN-y in serum, especially compound IA-3 had the most significant effect on the reduction of the above factors, and the results are shown in Fig. 3.

Histopathological examination was performed on the administration group of compound IA-3: mouse lungs were fixed with 4% paraformaldehyde, and histopathological changes were observed by HE. The results showed that the LPS group had severe lung injury, manifested by inflammatory cell infiltration, thickening of alveolar walls, pulmonary interstitial congestion, and bleeding. However, treatment with compound IA-3 reduced the severity of lung pathology. The results of H&E staining of mouse lung tissues were shown in Fig. 4.

### Example 5 Experimental study on inhibition of CD-3 antibody-induced cytokine storm by compounds

A total of 15 healthy male mice were randomly and equally divided into 3 groups, namely, control group, model group and drug administration group, with 5 mice in each group, labeled and their body weight recorded. Respectively administered: the control group and the model group were injected with normal saline via tail vein respectively, and the administration group was injected with the compounds prepared in Examples 1-2 via tail vein respectively; the dose was 10 mg/kg (2 mg/mL; 50 µL/10g). CD-3 antibody was injected intraperitoneally, 100 µg/kg, after 0.5 hours. A second injection of the drug was given 5 hours later. Blood was collected from the mice 9 hours later, followed by euthanasia.

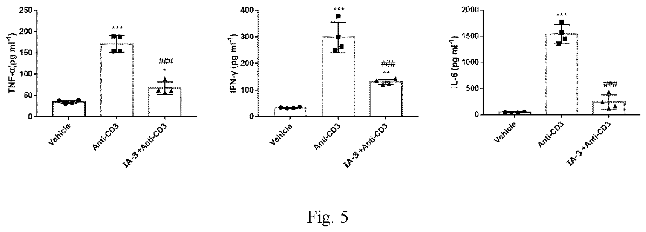

ELISA was performed to determine levels of IL-6, IL-2, TNF-a and IFN-y in serum. The results showed that the compounds of the present application exhibited different degrees of reduction in the levels of CD-3 antibody-induced inflammatory factors IL-6, IL-2, TNF-a and IFN-y in serum, especially compound IA-3 had the most significant effect on the reduction of the above factors, and the results are shown in Fig. 5.

### Example 6 Oral Bioavailability Study of Compounds

After experimental verification that the compounds of the present application have good oral bioavailability, this example uses compound IA-3, which exhibits relatively good inhibitory activity on immune cell proliferation, as an example, to illustrate the process of studying its oral bioavailability prior to its further study as an immunosuppressant in animal models, as follows:

### Instrument: Thermo Accela High Performance Liquid Chromatography System

To prepare the standard series solution, a suitable amount of compound IA-3 was accurately weighed and placed in a 100 mL volumetric flask. The compound was dissolved in acetonitrile and diluted to the mark to produce a 25.0 µg/mL stock solution. The solution was mixed thoroughly by shaking and used for further analysis. An appropriate amount of the stock solution was taken separately, diluted with acetonitrile, and made up to the mark to prepare a series of standard solutions with concentrations of 5, 10, 25, 50, 100, 250, 500, and 1000 ng/mL. The solutions were stored in a refrigerator at 4 °C for later use.

Preparation of internal standard solution: a suitable amount of internal standard was accurately weighed and placed in a 100 mL volumetric flask. The internal standard was dissolved in acetonitrile and diluted to the mark to produce a 25 µg/mL stock solution. The solution was mixed thoroughly by shaking and used for further analysis. An accurately weighed amount of the stock solution was taken again and diluted with acetonitrile to prepare the internal standard reference solution at a concentration of 50 ng/mL. The solution was stored in a refrigerator at 4 °C for later use.

Preparation of plasma samples: 80 µL of rat plasma was taken, and 120 µL of internal standard was added. The mixture was vortexed for 2 min and centrifuged for 10 min. Then, 140 µL of the supernatant was collected and centrifuged for another 10 min. Finally, 10 µL of the supernatant was injected for analysis.

Standard Curve: 64 µL of blank rat plasma was mixed with 16 µL of the test compound standard series solution and 120 µL of acetonitrile solution containing the internal standard. The mixture was vortexed for 2 minutes and centrifuged for 10 minutes. Then, 140 µL of the supernatant was taken and centrifuged for another 10 minutes, 10 µL of the sample was injected and analyzed with HPLC-HR-MS. Instrumentation obtained the linear regression equation as the standard curve.

Sampling: eight healthy male Wistar rats were randomly divided into two groups (oral group and intravenous group), with four rats in each group. Rats were fasted for 12 h and provided with free access to water before the experiment. The oral group was administered with the test drug at a dose of 5 mg/kg, and the intravenous group was administered with 1 mg/kg. Blood samples of approximately 0.4 mL were collected from the rat orbital vein before and at 5 min, 15 min, 30 min, 1 h, 1.5 h, 2 h, 4 h, 6 h, 8 h, 10 h, and 12 h after administration, and immediately transferred to heparinized centrifuge tubes. Plasma was separated by centrifugation.

Sample determination and data processing: the rat plasma samples were processed according to the "Preparation of plasma samples" procedure and analyzed under the conditions described above. The non-compartmental model was applied to process the drug concentration data mentioned above, and the pharmacokinetic parameters were calculated using the DAD 2.0 software. The bioavailability was calculated according to the formula F=AUC_{ig}*Dᵢᵥ/ AUCᵢᵥ*D_{ig}*100%.

The results were shown in Tables 5 and 6:

**Table 5. Pharmacokinetics after single injection at a dose of 1 mg/kg**

| Compound | AUC0-t (h*ng/mL) | AUC0-∞ (h*ng/mL) | MRT (h) | t_{1/2} (h) | CL (mL/min/kg) | Vss (l/kg) |
|---|---|---|---|---|---|---|
| IA-3 | 717.35 | \ | 0.506 | 0.36 | 45.1 | 1.08 |

| Table 6. Pharmacokinetics after single oral administration at a dose of 5 mg/kg | | | | | | |
|---|---|---|---|---|---|---|
| Compound | AUC0-t (h*ng/mL) | AUC0-∞ (h*ng/mL) | MRT (h) | t_{1/2} (h) | CL (mL/min/kg) | Vss (l/kg) |
| IA-3 | 407.74 | 407.74 | 1.27 | 2.09 | 384 | 69.5 |

After intragastric administration of IA-3, the total exposure (AUC) in whole blood was higher, with a relative oral bioavailability of 22.07%. These results suggest that IA-3 has the potential for becoming a drug.

## Claims

1. A compound of formula X or pharmaceutically acceptable salt thereof: 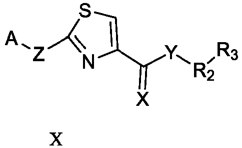 wherein A is a struture of pyrazolopyrimidine or indole and the compound conforms to a structure of Formula X₁: 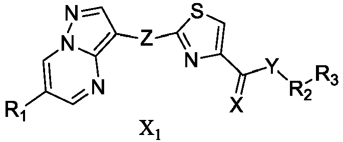
Z is absent or carbonyl;
X is O or S;
Y is -O-, -NH- or 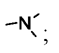 ;
R₁ is hydrogen or C₁-C₆ alkyl;
R₂ is selected from C₁-C₃ alkyl, C₅-C₁₅ alkenyl, alkynyl, 5-10 membered heterocyclyl, C₆-C₁₂ aryl, 5-12 membered heteroaryl, sterol group and 5-10 membered cycloalkyl; Y and R₂ are directly connected, or Y and R₂ are connected to form a ring;
R₃ is selected from hydrogen, halogen, amino, hydroxyl, acetyl, 3-10 membered heterocyclyl, C₆-C₁₂ aryl, 5-12 membered heteroaryl, 3-10 membered cycloalkyl, ester group, carboxyl, trihalomethyl and adamantyl;
R₂ or R₃ is unsubstituted or is substituted by one or more groups selected from C₁-C₆ alkyl, hydroxyl, halogen, trihalomethyl, carboxyl and phenyl;
wherein R₃ is not hydrogen when R₂ is C₁-C₃ alkyl.

2. The compound or pharmaceutically acceptable salt thereof according to claim 1, wherein R₂ is selected from C₁-C₃ alkyl, C₅-C₁₅ monoalkenyl, C₅-C₁₅ dienyl, C₅-C₁₅ trienyl, alkynyl, 5-6 membered cycloalkyl, phenyl, 5-6 membered heterocyclyl, 5-6 membered heteroaryl and sterol group; wherein Y and R₂ are directly connected, or Y and R₂ are connected to form a ring;
R₂ is unsubstituted or is substituted by one or more groups selected from C₁-C₆ alkyl, hydroxyl, halogen, trihalomethyl and carboxyl;
preferably, R₃ is selected from hydrogen, halogen, amino, hydroxyl, acetyl, 5-6 membered heterocyclyl, phenyl, biphenyl, naphthyl, 5-6 membered heteroaryl, 5-6 membered cycloalkyl, ester group, carboxyl, amido, trihalomethyl and adamantyl;
R₃ is unsubstituted or is substituted by one or more groups selected from C₁-C₆ alkyl, hydroxyl, halogen, trihalomethyl, carboxyl and phenyl.

3. The compound or pharmaceutically acceptable salt thereof according to claim 1, wherein R₂ is selected from methyl, ethyl, propyl, C₅ monoalkenyl, C₁₀ dienyl, C₁₅ trienyl, alkynyl, cyclopentyl, cyclohexyl, triazolyl, phenyl, piperidinyl, piperazinyl, pyrrolidinyl, pyridyl, pyrimidyl, sterol group; wherein Y and R₂ are directly connected, or Y and R₂ are connected to form a ring;
R₂ is unsubstituted or is substituted by one or more groups selected from C₁-C₆ alkyl, hydroxyl, halogen, trihalomethyl and carboxyl;
wherein the sterol group is selected from 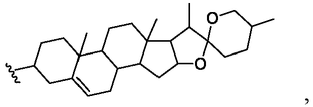 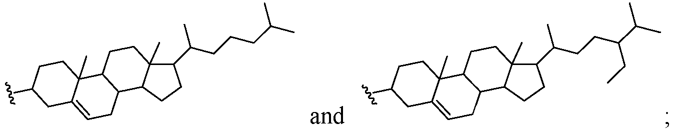
preferably, R₃ is selected from hydrogen, halogen, amino, hydroxyl, acetyl, phenyl, biphenyl, naphthyl, cyclopentyl, cyclohexyl, piperidinyl, piperazinyl, pyrrolidinyl, pyridyl, pyrimidyl, ester group, carboxyl, amido, trihalomethyl and adamantyl;
R₃ is unsubstituted or is substituted by one or more groups selected from C₁-C₆ alkyl, hydroxyl, halogen, trihalomethyl, carboxyl and phenyl.

4. The compound or pharmaceutically acceptable salt thereof according to any one of claims 1 to 3, wherein the compound has a structure of Formula I: 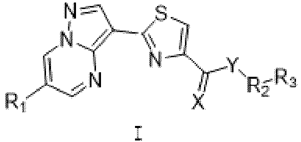
wherein X, Y, R₁, R₂ and R₃ are each independently the same as defined in any one of claims 1 to 3;
preferably, in the compound of Formula I,
X is O or S;
Y is -O-, -NH- or 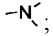
R₁ is hydrogen or C₁-C₂ alkyl;
R₂ is selected from methyl, ethyl, C₅ monoalkenyl, C₁₀ dienyl, cyclohexyl, phenyl and pyridyl; wherein Y and R₂ are directly connected, or Y and R₂ are connected to form a ring;
R₃ is selected from hydrogen, phenyl, pyridyl, pyrimidyl, ester group, trihalomethyl;
R₂ or R₃ is unsubstituted, or is substituted by one or more groups selected from C₁-C₆ alkyl, hydroxyl, halogen, trihalomethyl and carboxyl;
preferably, in the compound of Formula II,
X is O or S;
Y is -O-, -NH- or 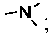
R₂ is selected from methyl, ethyl, propyl, C₅ monoalkenyl, C₁₀ dienyl, C15 trienyl, alkynyl, cyclopentyl, cyclohexyl, phenyl, triazolyl, pyridyl and sterol group; wherein Y and R₂ are directly connected, or Y and R₂ are connected to form a ring;
wherein the sterol group is selected from 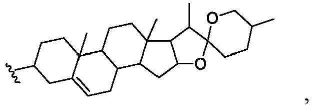 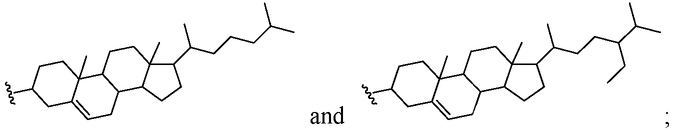
R₃ is selected from hydrogen, halogen, amino, hydroxyl, acetyl, phenyl, biphenyl, naphthyl, cyclopentyl, cyclohexyl, pyrrolidinyl, pyridyl, pyrimidyl, ester group, carboxyl, amido, trihalomethyl and adamantyl;
R₂ or R₃ is unsubstituted or is substituted by one or more groups selected from C₁-C₆ alkyl, hydroxyl, halogen, trihalomethyl and carboxyl.

5. The compound or pharmaceutically acceptable salt thereof according to claim 4, wherein X is O, and the compound has a structure of Formula IA: 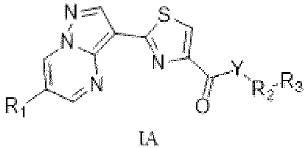
wherein Y, R₁, R₂ and R₃ are each independently the same as defined in any one of claims 1 to 4; Y and R₂ are directly connected, or Y and R₂ are connected to form a ring;
preferably, X is S, and the compound has a structure of Formula IB: 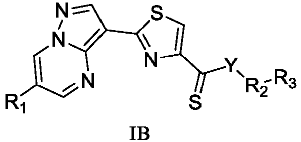
wherein Y is -NH-, R₁, R₂ and R₃ are each independently the same as defined in any one of claims 1 to 4;
preferably, in the compound of Formula IB, R₂ is selected from methyl, ethyl and pyridyl; R₃ is selected from hydrogen, pyridyl and pyrimidyl.

6. The compound or pharmaceutically acceptable salt thereof according to claim 5, wherein Y is -O-, -NH- or 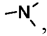 and X is O;
wherein, Y is -O- or -NH-, Y and R₂ are directly connected; or
when Y is 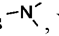 Y and R₂ are connected to born a cyclic R₂' structure, and the N atom is a ring-forming atom on the R₂' structure;
preferably, the compound has a structure of Formula IA₁, IA₂, or IA₃: 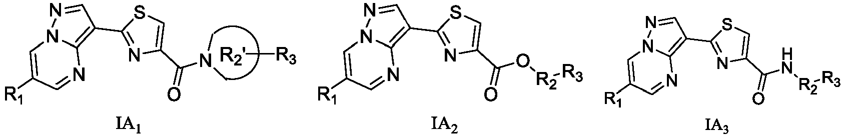
wherein R₁, R₂ and R₃ are each independently the same as defined in any one of claims 1 to 5; R₂' is selected from 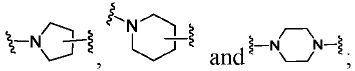
preferably, in the compound of Formula IIA₂, R₂ is selected from 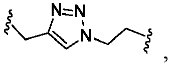 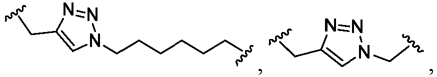
and R₃ is selected from halogen, hydroxyl, phenyl, naphthyl and adamantyl.

7. The compound or pharmaceutically acceptable salt thereof according to any one of claims 1 to 6, wherein the compound is selected from the following structures:

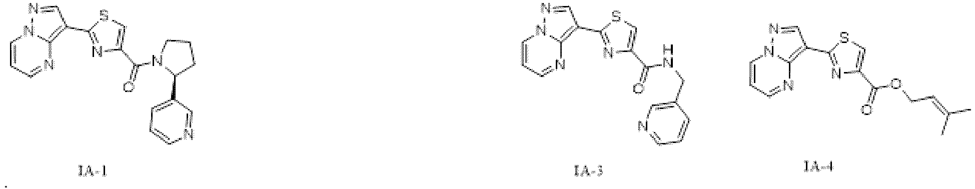

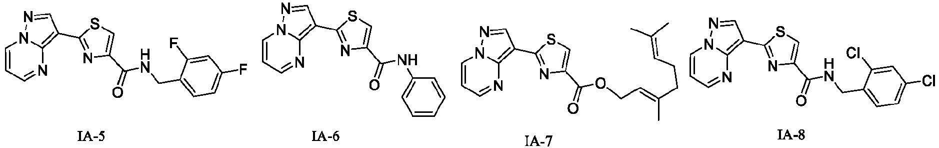

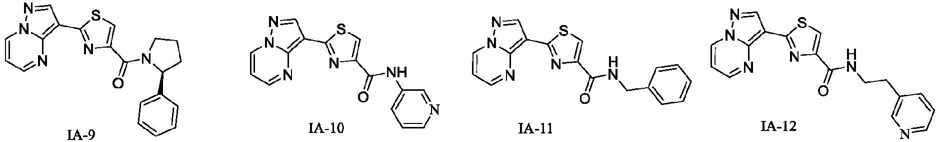

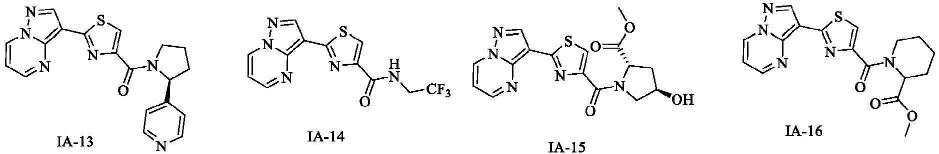

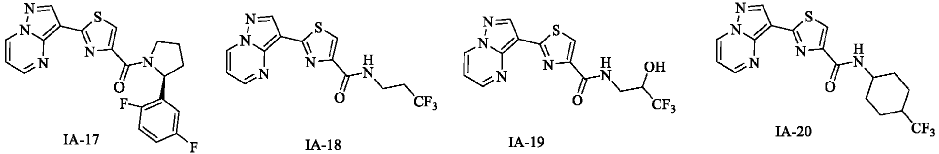

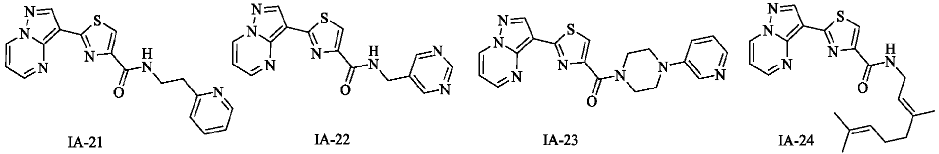

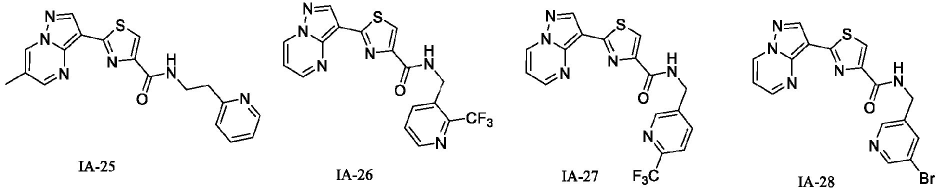

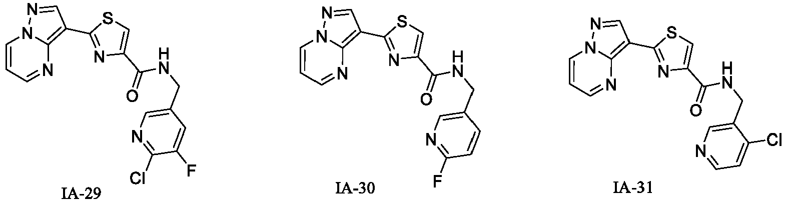

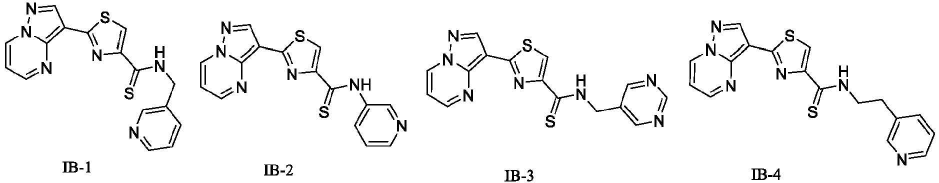

8. A method for preparing a compound or pharmaceutically acceptable salt thereof according to any one of claims 1 to 7, comprising:
cyclizing compound 1 with a compound 2 to obtain a compound 3; hydrolyzing the ester bond of the compound 3 to obtain a compound 4; acyl-chlorinating and aminating the compound 4 to obtain a compound 5; substituting the compound 5 with sulfur to obtain a compound 6; cyclizing the compound 6 to obtain a compound 8; hydrolyzing the ester bond of the compound 8 to obtain a compound 9; performing amide condensation or ester condensation between the compound 9 and a compound 10 to obtain a compound of Formula IA;
alternatively, preparing a compound of Formula IB by oxidation sulfur exchange of a compound of formula IA;
wherein compounds 1-10 are as follows: 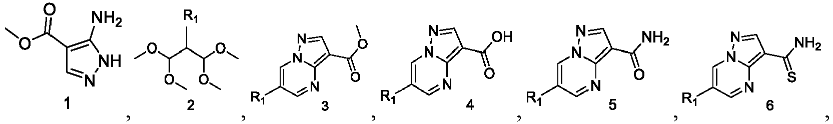 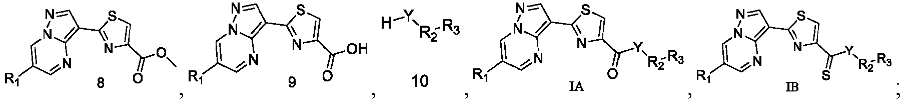 wherein R₁, R₂, R₃ and Y are each independently the same as defined in any one of claims 1 to 7.

9. Pharmaceutical composition or pharmaceutical formulation, comprising at least a compound or pharmaceutically acceptable salt thereof according to any one of claims 1 to 7;
alternatively, the pharmaceutical composition or pharmaceutical formulation further comprises at least a pharmaceutically acceptable excipient or a pharmaceutical carrier.

10. Compounds or pharmaceutically acceptable salts thereof according to any one of claims 1 to 7, or a pharmaceutical composition or pharmaceutical formulation according to claim 9, for use in the prevention and/or treatment of diseases or conditions related to anti-activation of immune system;
alternatively, a compound or pharmaceutically acceptable salts thereof according to any one of claims 1 to 7, or a pharmaceutical composition or pharmaceutical formulation according to claim 9, for use as immunosuppressive drugs;
preferably, the disease or condition is selected from the group consisting of rejection of organ, tissue or cell transplantation, graft-versus-host disease caused by transplantation, autoimmune syndrome, and diseases or conditions associated with cytokine storm;
preferably, the autoimmune syndrome includes lupus, systemic lupus erythematosus, psoriasis, eczema, dermatitis, arthritis, rheumatoid arthritis, spinal arthritis, gouty arthritis or other arthritic conditions, multiple sclerosis, dermatomycosis, antiphospholipid antibody syndrome, struma lymphomatosa, lymphocytic thyroiditis, multiple sclerosis, myasthenia gravis, type 1 diabetes, mellitus, uveitis, episcleritis, scleritis, Kawasaki's disease, uveoretinitis, choroiditis, uveitis associated with Behcet's syndrome, uveoencephalitis, viral encephalomyelitis, chronic allograft vascularopathy, post-infectious autoimmune diseases, rheumatic fever and post-infectious glomerulonephritis, inflammatory and cytoplastic dermatosis, psoriasis, psoriatic arthritis, atopic dermatitis, myopathy, myositis, osteomyelitis, contact dermatitis, dermatitis eczematosa, seborrheic dermatitis, lichen planus, pemphigus, urticaria, angioedema, angiitis, rubeola, acne vulgaris and mast-cell disease;
preferably, the disease or condition associated with cytokine storm is cytokine storm syndrome caused by an infectious disease.

## Patentansprüche

1. Verbindung der Formel X oder ein pharmazeutisch annehmbares Salz davon: 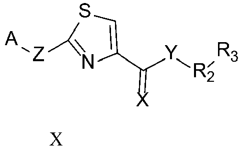 wobei A eine Struktur von Pyrazolopyrimidin oder Indol ist und die Verbindung einer Struktur der Formel X₁ entspricht: 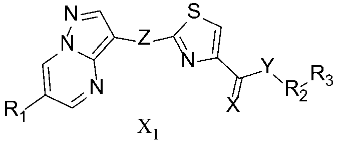
Z nicht vorhanden oder Carbonyl ist;
X O oder S ist;
Y -O-, -NH- oder 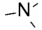 ist;
R₁ Wasserstoff oder C₁-C₆-Alkyl ist;
R₂ aus C₁-C₃-Alkyl, C₅-C₁₅-Alkenyl, Alkinyl, 5-10-gliedrigen Heterocyclyl-, C₆-C₁₂-Aryl-, 5-12-gliedrigen Heteroaryl-, Sterolgruppen und 5-10-gliedrigen Cycloalkylgruppen ausgewählt ist; Y und R₂ direkt verbunden sind oder Y und R₂ unter Bildung eines Rings verbunden sind;
R₃ aus Wasserstoff, Halogen, Amino, Hydroxyl, Acetyl, 3-10-gliedrigem Heterocyclyl, C₆-C₁₂-Aryl, 5-12-gliedrigem Heteroaryl, 3-10-gliedrigem Cycloalkyl, Estergruppe, Carboxyl, Trihalogenmethyl und Adamantyl ausgewählt ist;
R₂ oder R₃ unsubstituiert ist oder durch eine oder mehrere Gruppen substituiert ist, ausgewählt aus C₁-C₆-Alkyl, Hydroxyl, Halogen, Trihalogenmethyl, Carboxyl und Phenyl;
wobei R₃ kein Wasserstoff ist, wenn R₂ C₁-C₃-Alkyl ist.

2. Verbindung oder pharmazeutisch annehmbares Salz davon nach Anspruch 1, wobei R₂ aus C₁-C₃-Alkyl, C₅-C₁₅-Monoalkenyl, C₅-C₁₅-Dienyl, C₅-C₁₅-Trienyl, Alkinyl, 5-6-gliedrigem Cycloalkyl, Phenyl-, 5-6-gliedrigen Heterocyclyl-, 5-6-gliedrigen Heteroaryl- und Sterolgruppen ausgewählt ist; wobei Y und R₂ direkt verbunden sind oder Y und R₂ unter Bildung eines Rings verbunden sind;
R₂ unsubstituiert ist oder durch eine oder mehrere Gruppen substituiert ist, ausgewählt aus C₁-C₆-Alkyl, Hydroxyl, Halogen, Trihalogenmethyl und Carboxyl;
vorzugsweise R₃ aus Wasserstoff, Halogen, Amino, Hydroxyl, Acetyl, 5-6-gliedrigem Heterocyclyl, Phenyl, Biphenyl, Naphthyl, 5-6-gliedrigem Heteroaryl, 5-6-gliedrigem Cycloalkyl, Estergruppe, Carboxyl, Amido, Trihalomethyl und Adamantyl ausgewählt ist;
R₃ unsubstituiert ist oder durch eine oder mehrere Gruppen substituiert ist, ausgewählt aus C₁-C₆-Alkyl, Hydroxyl, Halogen, Trihalogenmethyl, Carboxyl und Phenyl;

3. Verbindung oder pharmazeutisch annehmbares Salz davon nach Anspruch 1, wobei R₂ aus Methyl, Ethyl, Propyl, C₅-Monoalkenyl, C₁₀-Dienyl, C₁₅-Trienyl, Alkinyl, Cyclopentyl, Cyclohexyl, Triazolyl, Phenyl-, Piperidinyl-, Piperazinyl-, Pyrrolidinyl-, Pyridyl-, Pyrimidyl-, Sterolgruppe ausgewählt ist; wobei Y und R₂ direkt verbunden sind oder Y und R₂ unter Bildung eines Rings verbunden sind;
R₂ unsubstituiert ist oder durch eine oder mehrere Gruppen substituiert ist, ausgewählt aus C₁-C₆-Alkyl, Hydroxyl, Halogen, Trihalogenmethyl und Carboxyl;
wobei die Sterolgruppe aus 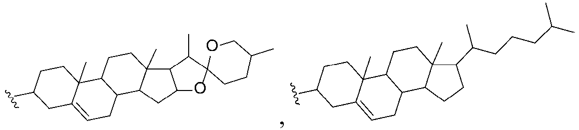 und 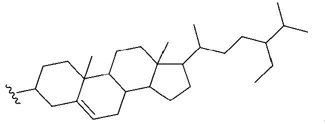 ausgewählt ist;
vorzugsweise R₃ aus Wasserstoff, Halogen, Amino, Hydroxyl, Acetyl, Phenyl, Biphenyl, Naphthyl, Cyclopentyl, Cyclohexyl, Piperidinyl, Piperazinyl, Pyrrolidinyl, Pyridyl, Pyrimidyl, Estergruppe, Carboxyl, Amido, Trihalogenmethyl und Adamantyl ausgewählt ist;
R₃ unsubstituiert ist oder durch eine oder mehrere Gruppen substituiert ist, ausgewählt aus C₁-C₆-Alkyl, Hydroxyl, Halogen, Trihalogenmethyl, Carboxyl und Phenyl;

4. Verbindung oder pharmazeutisch annehmbares Salz davon nach einem der Ansprüche 1 bis 3, wobei die Verbindung eine Struktur der Formel I aufweist: 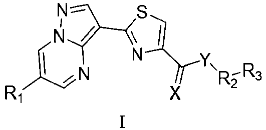
wobei X, Y, R₁, R₂ und R₃ jeweils unabhängig voneinander wie in einem der Ansprüche 1 bis 3 definiert sind;
vorzugsweise in der Verbindung der Formel I,
X O oder S ist;
Y -O-, -NH- oder 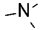 ist;
R₁ Wasserstoff oder C₁-C₂-Alkyl ist;
R₂ aus Methyl, Ethyl, C₅-Monoalkenyl, C₁₀-Dienyl, Cyclohexyl, Phenyl und Pyridyl ausgewählt ist; wobei Y und R₂ direkt verbunden sind oder Y und R₂ unter Bildung eines Rings verbunden sind;
R₃ ausgewählt ist aus Wasserstoff, Phenyl, Pyridyl, Pyrimidyl, Estergruppe, Trihalogenmethyl;
R₂ oder R₃ unsubstituiert ist oder durch eine oder mehrere Gruppen substituiert ist, ausgewählt aus C₁-C₆-Alkyl, Hydroxyl, Halogen, Trihalogenmethyl und Carboxyl;
vorzugsweise in der Verbindung der Formel II,
X O oder S ist;
Y -O-, -NH- oder 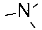 ist;
wobei R₂ aus Methyl, Ethyl, Propyl, C₅-Monoalkenyl, C₁₀-Dienyl, C₁₅-Trienyl, Alkinyl, Cyclopentyl, Cyclohexyl, Phenyl, Triazolyl, Pyridyl und Sterolgruppen ausgewählt ist; wobei Y und R₂ direkt verbunden sind oder Y und R₂ unter Bildung eines Rings verbunden sind;
wobei die Sterolgruppe aus 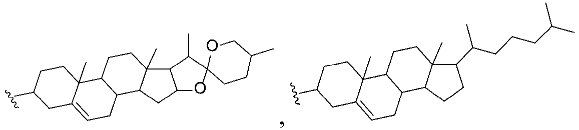 und 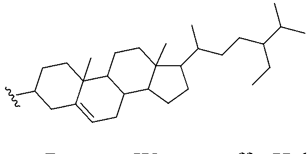 ausgewählt ist;
R₃ aus Wasserstoff, Halogen, Amino, Hydroxyl, Acetyl, Phenyl, Biphenyl, Naphthyl, Cyclopentyl, Cyclohexyl, Pyrrolidinyl, Pyridyl, Pyrimidyl, Estergruppe, Carboxyl, Amido, Trihalogenmethyl und Adamantyl ausgewählt ist;
R₂ oder R₃ unsubstituiert ist oder durch eine oder mehrere Gruppen substituiert ist, ausgewählt aus C₁-C₆-Alkyl, Hydroxyl, Halogen, Trihalogenmethyl und Carboxyl.

5. Verbindung oder pharmazeutisch annehmbares Salz davon nach Anspruch 4, wobei X O ist und die Verbindung eine Struktur der Formel IA aufweist: 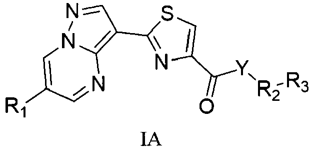
wobei Y, R₁, R₂ und R₃ jeweils unabhängig voneinander wie in einem der Ansprüche 1 bis 4 definiert sind; Y und R₂ direkt verbunden sind oder Y und R₂ unter Bildung eines Rings verbunden sind;
vorzugsweise X S ist und die Verbindung eine Struktur der Formel IB aufweist: 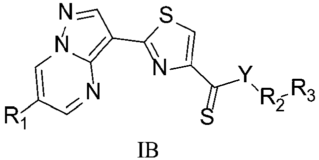
wobei Y -NH- ist, R₁, R₂ und R₃ jeweils unabhängig voneinander wie in einem der Ansprüche 1 bis 4 definiert sind;
vorzugsweise in der Verbindung der Formel IB, R₂ aus Methyl, Ethyl und Pyridyl ausgewählt ist; R₃ aus Wasserstoff, Pyridyl und Pyrimidyl ausgewählt ist.

6. Verbindung oder pharmazeutisch annehmbares Salz davon nach Anspruch 5, wobei Y -O-, -NH- oder 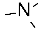 ist; und X O ist;
wobei Y -O- oder -NH- ist, Y und R₂ direkt verbunden sind; oder
wenn Y 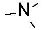 ist, Y und R₂ verbunden sind, so dass eine cyclische R₂'-Struktur entsteht, und das N-Atom an der R₂'-Struktur ein ringbildendes Atom ist;
vorzugsweise die Verbindung eine Struktur der FormelIA₁, IA₂, oder IA₃ aufweist: 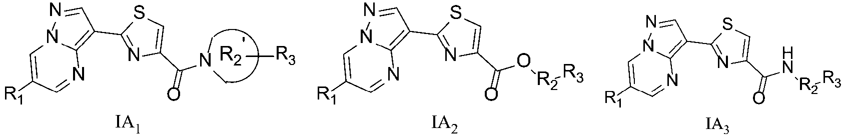
wobei R₁, R₂ und R₃ jeweils unabhängig voneinander wie in einem der Ansprüche 1 bis 5 definiert sind; R₂' aus 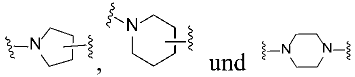 ausgewählt ist;
vorzugsweise in der Verbindung der Formel IIA₂ R₂ aus 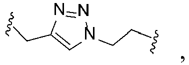 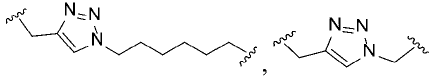 ausgewählt ist und R₃ aus Halogen, Hydroxyl, Phenyl, Naphthyl und Adamantyl ausgewählt ist.

7. Verbindung oder pharmazeutisch annehmbares Salz davon nach einem der Ansprüche 1 bis 6, wobei die Verbindung aus den folgenden Strukturen ausgewählt ist:

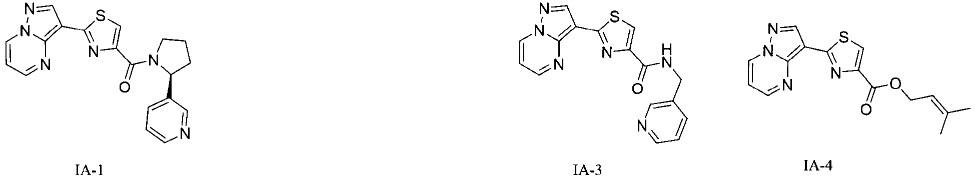

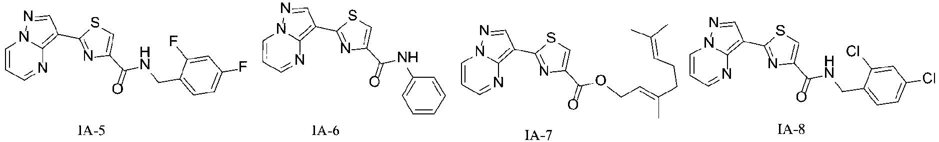

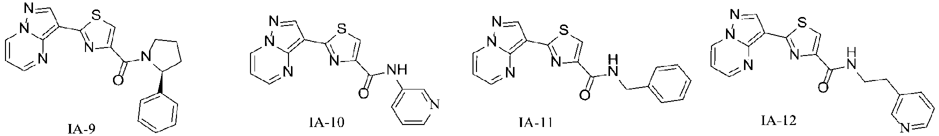

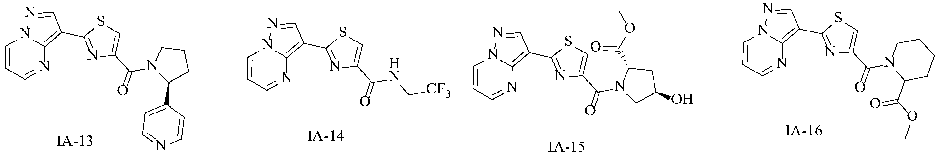

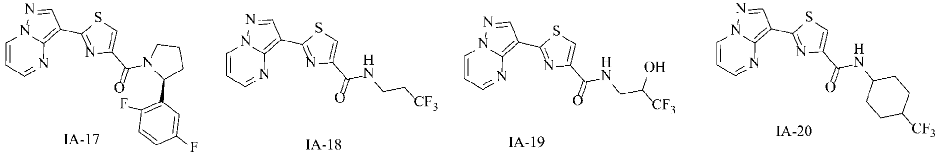

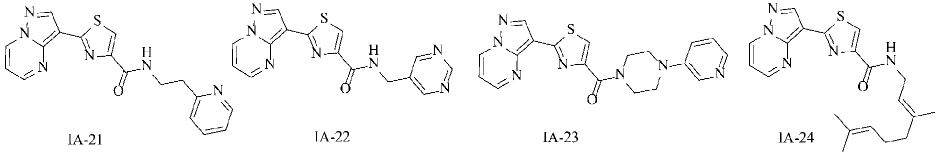

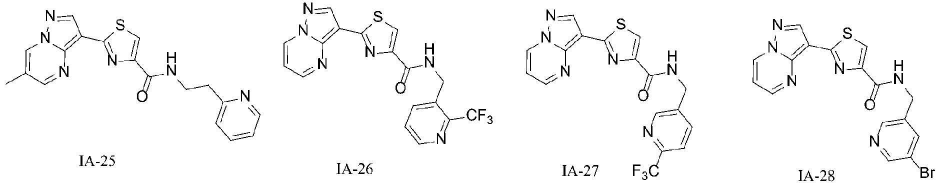

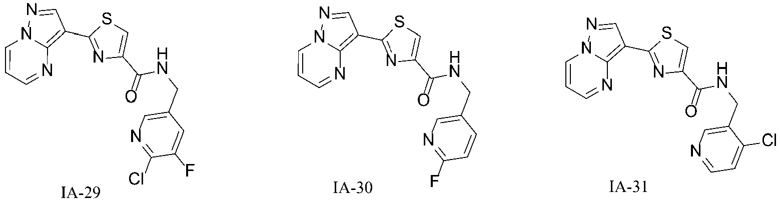

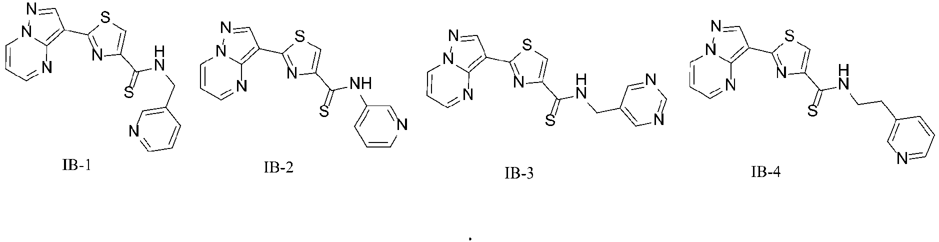

8. Verfahren zur Herstellung einer Verbindung oder eines pharmazeutisch annehmbaren Salzes davon nach einem der Ansprüche 1 bis 7, umfassend:
Zyklisierung der Verbindung 1 mit einer Verbindung 2, um eine Verbindung 3 zu erhalten; Hydrolysierung der Ester-Bindung der Verbindung 3, um eine Verbindung 4 zu erhalten; Acylchlorierung und Aminierung der Verbindung 4, um eine Verbindung 5 zu erhalten; Substituierung der Verbindung 5 mit Schwefel, um eine Verbindung 6 zu erhalten; Zyklisierung der Verbindung 6, um eine Verbindung 8 zu erhalten; Hydrolysierung der Ester-Bindung der Verbindung 8, um eine Verbindung 9 zu erhalten; Durchführung einer Amid-Kondensation oder Ester-Kondensation zwischen der Verbindung 9 und einer Verbindung 10, um eine Verbindung der Formel IA zu erhalten;
alternativ Herstellung einer Verbindung der Formel IB durch Oxidations-Schwefel-Austausch einer Verbindung der Formel IA;
wobei die Verbindungen 1-10 wie folgt sind: 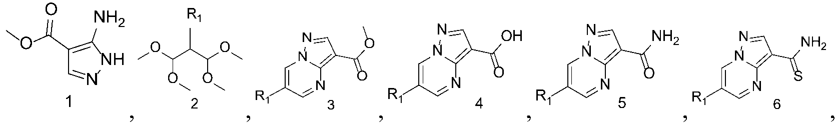 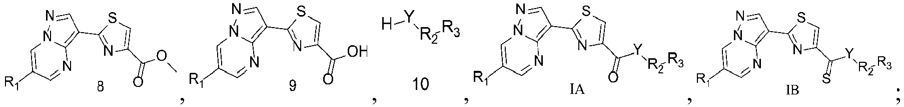 wobei R₁, R₂, R₃ und Y jeweils unabhängig voneinander wie in einem der Ansprüche 1 bis 7 definiert sind;

9. Pharmazeutische Zusammensetzung oder pharmazeutische Formulierung, umfassend mindestens eine Verbindung oder ein pharmazeutisch annehmbares Salz davon nach einem der Ansprüche 1 bis 7;
alternativ die pharmazeutische Zusammensetzung oder pharmazeutische Formulierung ferner mindestens einen pharmazeutisch annehmbaren Hilfsstoff oder einen pharmazeutischen Träger umfasst.

10. Verbindungen oder pharmazeutisch annehmbare Salze davon nach einem der Ansprüche 1 bis 7 oder eine pharmazeutische Zusammensetzung oder pharmazeutische Formulierung nach Anspruch 9 zur Verwendung bei der Vorbeugung und/oder Behandlung von Krankheiten oder Beschwerden im Zusammenhang mit der Anti-Aktivierung des Immunsystems;
alternativ eine Verbindung oder pharmazeutisch annehmbare Salze davon nach einem der Ansprüche 1 bis 7 oder eine pharmazeutische Zusammensetzung oder pharmazeutische Formulierung nach Anspruch 9 zur Verwendung als immunsuppressive Arzneimittel;
vorzugsweise die Krankheit oder die Beschwerden aus der Gruppe bestehend aus Abstoßung von Transplantaten von Organen, Geweben oder Zellen, durch Transplantation verursachter Graft-versus-Host-Krankheit, Autoimmunsyndrom und mit Zytokinsturm assoziierten Krankheiten oder Beschwerden ausgewählt sind;
wobei das Autoimmunsyndrom vorzugsweise Lupus, systemischen Lupus erythematodes, Psoriasis, Ekzem, Dermatitis, Arthritis, rheumatoide Arthritis, Spondylarthritis, Gichtarthritis oder andere arthritische Erkrankungen, Multiple Sklerose, Dermatomykose, Antiphospholipid-Antikörper-Syndrom, Struma lymphomatosa, lymphozytäre Thyreoiditis, Multiple Sklerose, Myasthenia gravis, Typ-1-Diabetes mellitus, Uveitis, Episkleritis, Skleritis, Kawasaki-Krankheit, Uveoretinitis, Choroiditis, mit dem Behcet-Syndrom assoziierte Uveitis, Uveoenzephalitis, virale Enzephalomyelitis, chronische Allotransplantat-Vaskulopathie, postinfektiöse AutoimmunErkrankungen, rheumatisches Fieber und postinfektiöse Glomerulonephritis, entzündliche und zytoplastische Dermatosen, Psoriasis, Psoriasis-Arthritis, atopische Dermatitis, Myopathie, Myositis, Osteomyelitis, Kontaktdermatitis, Dermatitis eczematosa, seborrhoische Dermatitis, Lichen planus, Pemphigus, Urtikaria, Angioödem, Angiitis, Rubeola, Akne vulgaris und Mastzellerkrankung einschließt;
vorzugsweise die mit dem Zytokinsturm assoziierte Krankheit oder Beschwerde das durch eine Infektionskrankheit verursachte Zytokinsturm-Syndrom ist.

## Revendications

1. Composé de formule X ou sel pharmaceutiquement acceptable de celui-ci : 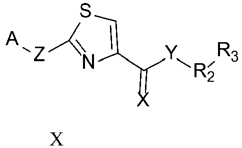 dans lequel A représente une structure de pyrazolopyrimidine ou d'indole et le composé est conforme à une structure de Formule X₁ : 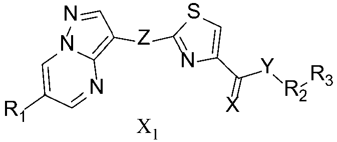
Z est absent ou représente carbonyle ;
X représente O ou S ;
Y représente -O-, -NH- ou 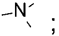
R₁ représente hydrogène ou alkyle C₁-C₆ ;
R₂ est choisi parmi alkyle C₁-C₃, alcényle C₅-C₁₅, alcynyle, hétérocyclyle à 5 à 10 chaînons, aryle C₆-C₁₂, hétéroaryle à 5 à 12 chaînons, groupe stérol et cycloalkyle à 5 à 10 chaînons ; Y et R₂ sont directement liés, ou Y et R₂ sont liés pour former un cycle ;
R₃ est choisi parmi hydrogène, halogène, amino, hydroxyle, acétyle, hétérocyclyle à 3 à 10 chaînons, aryle C6 à C12, hétéroaryle à 5 à 12 chaînons, cycloalkyle à 3 à 10 chaînons, groupe ester, carboxyle, trihalogénométhyle et adamantyle ;
R₂ ou R₃ est non substitué ou substitué par un ou plusieurs groupes choisis parmi alkyle C₁-C₆, hydroxyle, halogène, trihalogénométhyle, carboxyle et phényle ;
dans lequel R₃ ne représente pas hydrogène lorsque R₂ est alkyle C₁-C₃.

2. Composé ou sel pharmaceutiquement acceptable de celui-ci selon la revendication 1, dans lequel R₂ est choisi parmi alkyle C₁-C₃, monoalcényle C₅-C₁₅, diényle C₅-C₁₅, triényle C₅-C₁₅, alcynyle, cycloalkyle à 5 à 6 chaînons, phényle, hétérocyclyle à 5 à 6 chaînons, hétéroaryle à 5 à 6 chaînons et groupe stérol ; dans lequel Y et R₂ sont directement liés, ou Y et R₂ sont liés pour former un cycle ;
R₂ est non substitué ou substitué par un ou plusieurs groupes choisis parmi alkyle C₁-C₆, hydroxyle, halogène, trihalogénométhyle et carboxyle ;
de préférence, R₃ est choisi parmi hydrogène, halogène, amino, hydroxyle, acétyle, hétérocyclyle à 5 à 6 chaînons, phényle, biphényle, naphtyle, hétéroaryle à 5 à 6 chaînons, cycloalkyle à 5 à 6 chaînons, groupe ester, carboxyle, amido, trihalogénométhyle et adamantyle ;
R₃ est non substitué ou substitué par un ou plusieurs groupes choisis parmi alkyle C₁-C₆, hydroxyle, halogène, trihalogénométhyle, carboxyle et phényle ;

3. Composé ou sel pharmaceutiquement acceptable de celui-ci selon la revendication 1, dans lequel R₂ est choisi parmi méthyle, éthyle, propyle, monoalcényle C₅, diényle C₁₀, triényle C₁₅, alcynyle, cyclopentyle, cyclohexyle, triazolyle, phényle, pipéridinyle, pipérazinyle, pyrrolidinyle, pyridyle, pyrimidyle, groupe stérol ; dans lequel Y et R₂ sont directement liés, ou Y et R₂ sont liés pour former un cycle ;
R₂ est non substitué ou substitué par un ou plusieurs groupes choisis parmi alkyle C₁-C₆, hydroxyle, halogène, trihalogénométhyle et carboxyle ;
dans lequel le groupe stérol est choisi parmi 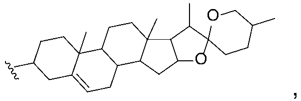 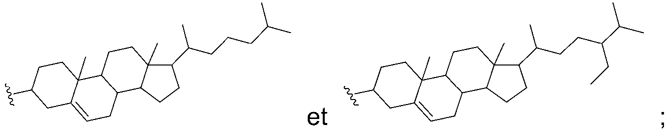
de préférence, R₃ est choisi parmi hydrogène, halogène, amino, hydroxyle, acétyle, phényle, biphényle, naphtyle, cyclopentyle, cyclohexyle, pipéridinyle, pipérazinyle, pyrrolidinyle, pyridyle, pyrimidyle, group ester, carboxyle, amido, trihalogénométhyle et adamantyle ;
R₃ est non substitué ou substitué par un ou plusieurs groupes choisis parmi alkyle C₁-C₆, hydroxyle, halogène, trihalogénométhyle, carboxyle et phényle ;

4. Composé ou sel pharmaceutiquement acceptable de celui-ci selon l'une quelconque des revendications 1 à 3, dans lequel le composé a une structure de Formule I : 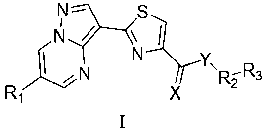
dans lequel X, Y, R₁, R₂ et R₃ sont chacun indépendamment identiques selon l'une quelconque des revendications 1 à 3 ;
de préférence, dans le composé de Formule I,
X représente O ou S ;
Y représente -O-, -NH- ou 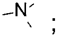
R₁ représente hydrogène ou alkyle C₁-C₂ ;
R₂ est choisi parmi méthyle, éthyle, monoalcényle C₅, diényle C₁₀, cyclohexyle, phényle et pyridyle ; dans lequel Y et R₂ sont directement liés, ou Y et R₂ sont liés pour former un cycle ;
R₃ est choisi parmi hydrogène, phényle, pyridyle, pyrimidyle, groupe ester, trihalogénométhyle ;
R₂ ou R₃ est non substitué ou substitué par un ou plusieurs groupes choisis parmi alkyle C₁-C₆, hydroxyle, halogène, trihalogénométhyle et carboxyle ;
de préférence, dans le composé de Formule II,
X représente O ou S ;
Y représente -O-, -NH- ou 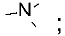
R₂ est choisi parmi méthyle, éthyle, propyle, monoalcényle C₅, diényle C₁₀, triényle C₁₅, alcynyle, cyclopentyle, cyclohexyle, phényle, triazolyle, pyridyle et groupe stérol ; dans lequel Y et R₂ sont directement liés, ou Y et R₂ sont liés pour former un cycle ;
dans lequel le groupe stérol est choisi parmi 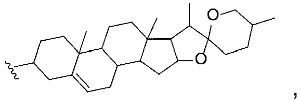 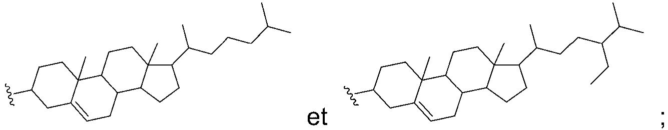
R₃ est choisi parmi hydrogène, halogène, amino, hydroxyle, acétyle, phényle, biphényle, naphtyle, cyclopentyle, cyclohexyle, pyrrolidinyle, pyridyle, pyrimidyle, group ester, carboxyle, amido, trihalogénométhyle et adamantyle ;
R₂ ou R₃ est non substitué ou substitué par un ou plusieurs groupes choisis parmi alkyle C₁-C₆, hydroxyle, halogène, trihalogénométhyle et carboxyle ;

5. Composé ou sel pharmaceutiquement acceptable de celui-ci selon la revendication 4, dans lequel X représente O, et le composé a une structure de Formule IA : 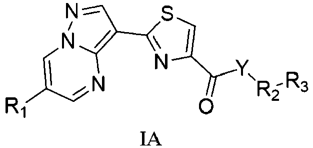
dans lequel Y, R₁, R₂ et R₃ sont chacun indépendamment identiques selon l'une quelconque des revendications 1 à 4 ; Y et R₂ sont directement liés, ou Y et R₂ sont liés pour former un cycle ;
de préférence, X représente S, et le composé a une structure de Formule IB : 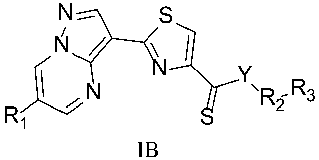
dans lequel Y représente -NH-, R₁, R₂ et R₃ sont chacun indépendamment identiques selon l'une quelconque des revendications 1 à 4 ;
de préférence, dans le composé de Formule IB, R₂ est choisi parmi méthyle, éthyle et pyridyle ; R₃ est choisi parmi hydrogène, pyridyle et pyrimidyle.

6. Composé ou sel pharmaceutiquement acceptable de celui-ci selon la revendication 5, dans lequel Y représente -O-, -NH- ou 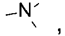 et X représente O ;
dans lequel, Y représente -O- ou -NH-, Y et R₂ sont directement liés ; ou
lorsque Y représente 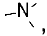 Y et R₂ sont liés pour former une structure R₂' cyclique, et l'atome N est un atome formant un cycle sur la structure R₂' ;
de préférence, le composé a une structure de Formule IA₁, IA₂, ou IA₃ : 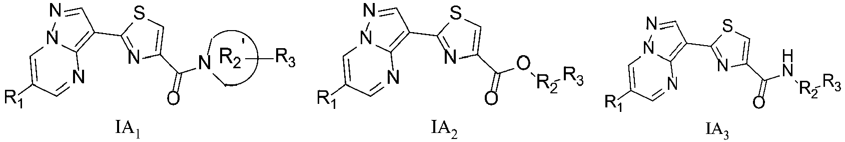
dans lequel R₁, R₂ et R₃ sont chacun indépendamment identiques selon l'une quelconque des revendications 1 à 5 ; R₂' est choisi parmi 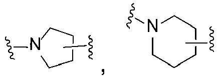
et 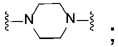
de préférence, dans le composé de Formule IIA₂, R₂ est choisi parmi 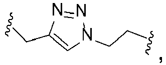 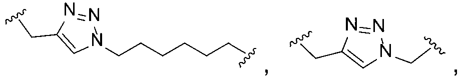 et R₃ est choisi parmi halogène, hydroxyle, phényle, naphtyle et adamantyle.

7. Composé ou sel pharmaceutiquement acceptable de celui-ci selon l'une quelconque des revendications 1 à 6, dans lequel le composé est choisi parmi les structures suivantes :

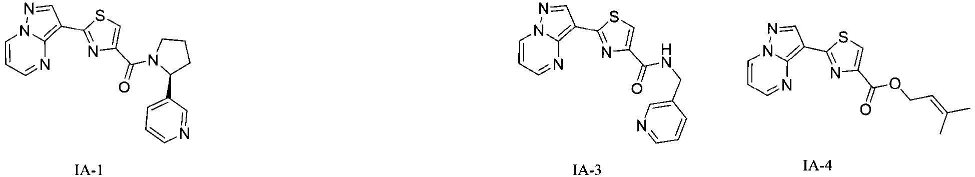

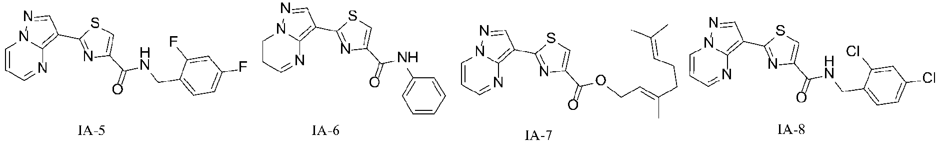

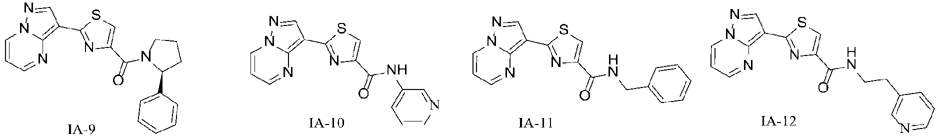

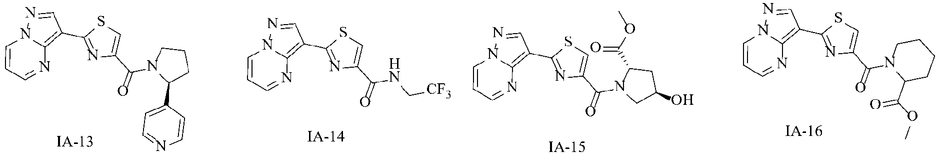

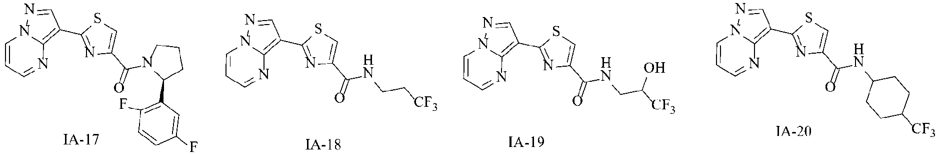

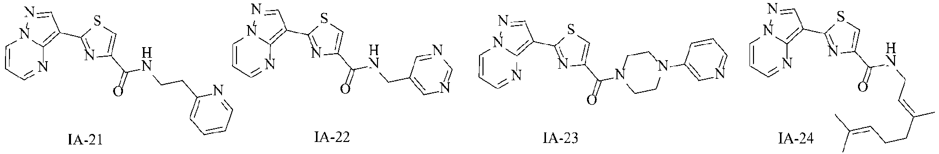

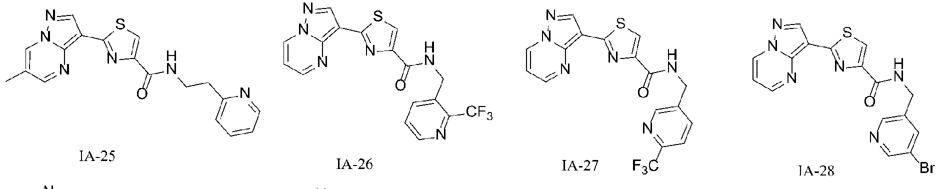

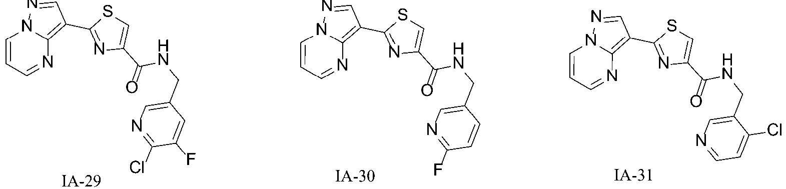

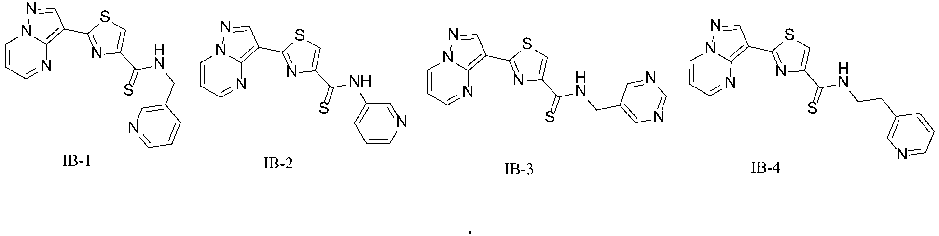

8. Procédé de préparation d'un composé ou d'un sel pharmaceutiquement acceptable de celui-ci selon l'une quelconque des revendications 1 à 7, comprenant les étapes consistant à :
cycliser un composé 1 avec un composé 2 afin d'obtenir un composé 3 ; hydrolyser la liaison ester du composé 3 afin d'obtenir un composé 4 ; acyl-chloriner et aminer le composé 4 afin d'obtenir un composé 5 ; substituer le composé 5 par du soufre afin d'obtenir un composé 6 ; cycliser le composé 6 afin d'obtenir un composé 8 ; hydrolyser la liaison ester du composé 8 afin d'obtenir un composé 9 ; effectuer une condensation d'amide ou une condensation d'ester entre le composé 9 et un composé 10 afin d'obtenir un composé de Formule IA ;
alternativement, préparer un composé de Formule IB par échange oxygène-soufre d'un composé de Formule IA ;
dans lequel les composés 1 à 10 sont les suivants : 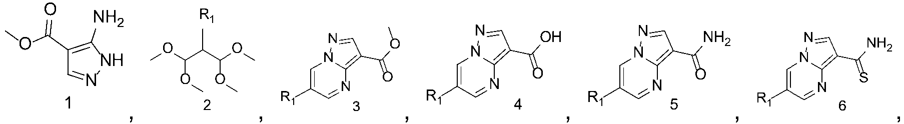 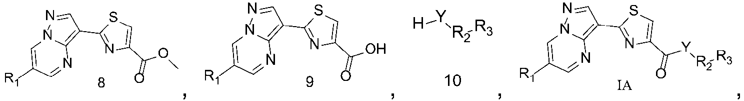 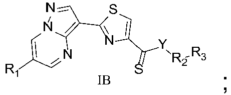 dans lequel R₁, R₂, R₃ et Y sont chacun indépendamment identiques selon l'une quelconque des revendications 1 à 7.

9. Composition pharmaceutique ou formulation pharmaceutique, comprenant au moins un composé ou un sel pharmaceutiquement acceptable de celui-ci selon l'une quelconque des revendications 1 à 7 ;
alternativement, la composition pharmaceutique ou la formulation pharmaceutique comprend en outre au moins un excipient pharmaceutiquement acceptable ou un support pharmaceutique.

10. Composés ou sels pharmaceutiquement acceptables de ceux-ci selon l'une quelconque des revendications 1 à 7, ou composition pharmaceutique ou formulation pharmaceutique selon la revendication 9, pour une utilisation dans la prévention et/ou le traitement de maladies ou d'affections liées à l'anti-activation d'un système immunitaire ;
alternativement, un composé ou des sels pharmaceutiquement acceptables de celui-ci selon l'une quelconque des revendications 1 à 7, ou une composition pharmaceutique ou une formulation pharmaceutique selon la revendication 9, pour une utilisation comme médicaments immunosuppresseurs ;
de préférence, la maladie ou l'affection est choisie dans le groupe constitué d'un rejet d'organe, de tissu ou de greffe cellulaire, d'une maladie du greffon contre l'hôte causée par la transplantation, d'un syndrome auto-immun et de maladies ou affections associées à un choc cytokinique ;
de préférence, le syndrome auto-immun comporte le lupus, le lupus érythémateux disséminé, le psoriasis, l'eczéma, la dermatite, l'arthrite, la polyarthrite rhumatoïde, l'arthrite spinale, l'arthrite goutteuse ou d'autres affections arthritiques, la sclérose en plaques, la dermatomycose, le syndrome des anticorps antiphospholipides, le struma lymphomateux, la thyroïdite lymphocytaire, la sclérose en plaques, la myasthénie grave, le diabète de type 1, le diabète sucré, l'uvéite, l'épisclérite, la sclérite, la maladie de Kawasaki, l'uvéorétinite, la choroïdite, l'uvéite associée au syndrome de Behçet, l'uvéoencéphalite, l'encéphalomyélite virale, la vascularopathie chronique de l'allogreffe, les maladies auto-immunes post-infectieuses, le rhumatisme articulaire aigu et la glomérulonéphrite post-infectieuse, la dermatose inflammatoire et cytoplastique, le psoriasis, l'arthrite psoriasique, la dermatite atopique, la myopathie, la myosite, l'ostéomyélite, la dermatite de contact, la dermatite eczémateuse, la dermatite séborrhéique, le lichen plan, le pemphigus, l'urticaire, l'œdème de Quincke, l'angiite, la rubéole, l'acné vulgaire et la mastocytose ;
de préférence, la maladie ou l'affection associée au choc cytokinique est le syndrome de tempête de cytokines causé par une maladie infectieuse.
